(19) Europäisches Patentamt
European Patent Office
Office européen des brevets

(11) **EP 4 389 138 A1**

(12) **EUROPEAN PATENT APPLICATION**

(43) Date of publication:
**26.06.2024 Bulletin 2024/26**

(21) Application number: **23179636.8**

(22) Date of filing: **15.06.2023**

(51) International Patent Classification (IPC):
**A61K 38/20** (2006.01)   **A61P 1/16** (2006.01)
**A61P 3/04** (2006.01)   **A61P 3/10** (2006.01)
**A61K 47/54** (2017.01)

(52) Cooperative Patent Classification (CPC):
**A61K 38/20; A61K 47/542; A61P 1/16; A61P 3/04; A61P 3/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2022 EP 22215985**

(71) Applicant: **Cytoki Pharma ApS**
**2900 Hellerup (DK)**

(72) Inventors:
• **Skydsgaard, Karsten**
**2000 Frederiksberg (DK)**

• **Jørgensen, Rasmus**
**3500 Værløse (DK)**
• **Kjølbye, Anne Louise**
**2950 Vedbæk (DK)**
• **Van de Bunt, Gerrit Martinus**
**2300 København S (DK)**

(74) Representative: **EIP**
**Fairfax House**
**15 Fulwood Place**
**London WC1V 6HU (GB)**

Remarks:
The complete document including Reference Table(s) and the Sequence Listing(s) can be downloaded from the EPO website

(54) **DOSAGE REGIMEN FOR INTERLEUKIN-22 DERIVATIVES**

(57) The invention relates to derivatives of Interleukin-22 (IL-22), in particular those comprising a fatty acid covalently attached to an IL-22 protein, and a novel dosage regimen for treating metabolic, gut and liver diseases, disorders and conditions.

Figure 7

EP 4 389 138 A1

**Description**

**REFERENCE TO AN ELECTRONIC SEQUENCE LISTING**

The contents of the electronic sequence listing (CKTI-007/F01EP_SeqList_ST26.xml; Size: 46,749 bytes; and Date of Creation: 19 December 2022) are herein incorporated by reference in their entirety.

**FIELD OF THE INVENTION**

The present invention relates to derivatives of Interleukin-22 (IL-22), in particular those comprising a fatty acid covalently attached to an IL-22 protein, and a novel dosage regimen for treating metabolic, gut and liver diseases, disorders and conditions.

**BACKGROUND OF THE INVENTION**

In the human body, IL-22 is secreted as a response to cues reflecting pathogen infection and immune activation. The effect of IL-22 is the result of an orchestrated engagement of several activities/pathways. IL-22 acts on epithelial barrier tissues and organs upon injury to protect the cells and maintain barrier function. It also accelerates repair, prevents fibrosis and controls inflammation. IL-22 has been reported as able to treat a range of medical conditions, including pancreatitis, kidney failure, wounded skin and those often observed in diabetic or overweight mammals, such as hyperglycemia, hyperlipidemia and hyperinsulinemia.

However, IL-22 is generally cleared quickly from the body by the kidneys, which limits its use in clinical practice. This is a common feature of cytokines, and half-life extended cytokine drug development candidates have reached the drug development stage for treatment of e.g., oncology and immunotherapy. Generally, these half-life extended cytokines use Fc fusion solutions or PEGylations. Known methods for extending the half-life of circulating IL-22 therefore seek to artificially increase the size of IL-22 beyond 70 kDa, so as to avoid renal clearance. Genentech and Generon Shanghai both have long-acting IL-22-Fc fusions in clinical development. Modifying IL-22 with polyethylene glycol (PEGylation) is another known means for avoiding renal clearance. However, these existing solutions are not without their disadvantages, which include immunogenicity, decreased activity and heterogeneity for PEGylation and poor distribution, receptor engagement kinetics and potency for Fc fusions.

A novel and improved class of IL-22 derivatives has been found and described in International Publication Nos. WO 2019/101888, WO 2022/238503 and WO 2022/238510. These biocompatible derivatives comprise a fatty acid covalently attached to an IL-22 protein. They enhance circulating half-lives and demonstrate optimised pharmacokinetic (PK) and pharmacodynamic (PD) properties compared to the native molecule. They maintain potency and other properties of the native molecule and also avoid toxicity, immunogenicity and other adverse reactions demonstrated by alternative derivatives of IL-22 such as the PEGylated derivatives and Fc fusions referred to above.

Moreover, these derivatives have been shown to be efficacious in the treatment and/or prevention of a range of diseases, disorders and conditions in animal models including diabetes, liver injury, lung injury, colitis, obesity and non-alcoholic steatohepatitis (NASH).

There nevertheless remains a need for therapeutically efficacious dosage regimens in man. It was thus an object of the present invention to find a dosage regimen that minimised adverse side effects without substantially compromising the beneficial effect of treatment, particularly in the treatment of metabolic, gut and liver diseases, disorders or conditions.

**SUMMARY OF THE INVENTION**

In a first aspect, there is provided a derivative of IL-22 comprising a fatty acid covalently attached to an IL-22 protein, for use in a method of treating a metabolic, gut and/or liver disease, disorder or condition, said method comprising administering the derivative of IL-22 subcutaneously.

In a second aspect, there is provided a derivative of IL-22 comprising a fatty acid covalently attached to an IL-22 protein, for use in a method of treating a metabolic, gut and/or liver disease, disorder or condition, said method comprising administering the derivative of IL-22 intravenously.

**BRIEF DESCRIPTION OF THE DRAWINGS**

Figure 1 illustrates a (A) C18 diacid, (B) C16 diacid, and (C) C14 diacid, each connected to a linker comprising a Cys-reactive unit. These combinations of fatty acids and linkers are employed in the derivatives identified herein as Derivatives 1-12.

Figure 2 illustrates the structure of a derivative identified herein as Derivative 1.

Figure 3 illustrates the structure of a derivative identified herein as Derivative 6.

Figure 4 illustrates the structure of a derivative identified herein as Derivative 10.

Figure 5 illustrates the structure of a derivative identified herein as Derivative 11.

Figure 6 illustrates the structure of a derivative identified herein as Derivative 12.

Figure 7 illustrates the reduction in colonic inflammation volume in a murine model of colitis after subcutaneous administration of Derivative 1 (see Table 7 for identification of mouse groups).

Figure 8 illustrates *in vitro* signal transducer and activator of transcription 3 (STAT3) assay curves for Derivative 1. The y-axis shows luminescence in relative light units (RLU) and the x-axis shows Derivative 1 concentration. "Repeat 01" and "Repeat 02" are two independent data sets obtained by repeating the assay.

Figure 9 illustrates the normalisation of blood glucose in a murine db/db diabetes model after subcutaneous administration of Derivative 1 (see Table 11 for identification of mouse groups).

Figure 10 illustrates the reduction in body weight in a murine diet-induced obesity model after subcutaneous administration of Derivative 6 (see Table 12 for identification of mouse groups).

Figure 11 illustrates the reduction in (A) body weight, and (B) fasting plasma insulin in a murine diet-induced obesity model after subcutaneous administration of Derivative 1 (see Table 12 for identification of mouse groups).

Figure 12 illustrates the reduction in plasma alanine aminotransaminase (ALT) levels in a murine inflammatory acute liver failure model after subcutaneous administration of Derivative 1 (see Table 13 for identification of mouse groups).

Figure 13 illustrates increases in the IL-22 target engagement biomarkers, (A) regenerating islet-derived protein 3a (REG3a), and (B) highly sensitive C-reactive protein (hsCRP), following a single subcutaneous administration of Derivative 1 in humans.

## DETAILED DESCRIPTION

In what follows, Greek letters are represented by their symbol rather than their written name. For example, $\alpha$ = alpha, $\beta$ = beta, $\epsilon$ = epsilon, $\gamma$ = gamma and $\mu$ = mu. Amino acid residues may be identified by their full name, three-letter code or one-letter code, all of which are fully equivalent.

In a first aspect, there is provided a derivative of IL-22 comprising a fatty acid covalently attached to an IL-22 protein, for use in a method of treating a metabolic, gut and/or liver disease, disorder or condition, said method comprising administering the derivative of IL-22 subcutaneously.

In a second aspect, there is provided a derivative of IL-22 comprising a fatty acid covalently attached to an IL-22 protein, for use in a method of treating a metabolic, gut and/or liver disease, disorder or condition, said method comprising administering the derivative of IL-22 intravenously.

## I. Derivatives of IL-22

The term "derivative of IL-22", as used herein, refers to an IL-22 protein having a covalently attached fatty acid. The term encompasses both derivatives in which the fatty acid is covalently attached to the IL-22 protein directly and those in which the covalent attachment is by a linker, which itself can be devised of various subunits.

The covalent attachment of fatty acids is a proven technology for half-life extension of peptides and proteins and is a way of subtending a fatty acid from the peptide or protein. It is known from marketed products for types 1 and 2 diabetes, such as insulins Levemir® (detemir) and Tresiba® (degludec), and glucagon-like peptide-1 (GLP-1) derivatives Victoza® (liraglutide) and Ozempic® (semaglutide).

Fatty acid attachment enables binding to albumin, thereby preventing renal excretion and providing some steric protection against proteolysis. Advantageously, it offers a minimal modification to IL-22 compared to Fc fusion or PEGylation. In this regard, whilst Fc fusion and PEGylation aim to increase the size of IL-22 beyond the threshold for renal clearance, derivatives comprising a fatty acid covalently attached to an IL-22 protein retain a small size similar to that of the IL-22 protein. Thus, as the fatty acid attachment is a minimal modification, the resultant derivative is believed to maintain native-like properties including distribution, diffusion rate and receptor engagement (binding, activation and trafficking) and minimise immunogenicity risk.

As above, fatty acid attachment has proven therapeutic efficacy in insulin and GLP-1 derivatives for diabetes. However, IL-22 is a very different protein in terms of its size, sequence and biological properties. It was therefore counterintuitive to the inventors at the time that fatty acids could be covalently attached to IL-22 whilst maintaining therapeutic effect. It was particularly surprising that such a minimal modification to IL-22 could result in high potency (identical or close to hIL-22) combined with a very long circulatory half-life.

The term, "IL-22 protein", as used herein, can mean a native IL-22 protein, such as hIL-22, or a variant thereof. A "variant" can be a protein having a similar amino acid sequence to that of the native protein, as further defined herein.

In nature, human IL-22 protein is synthesised with a signal peptide of 33 amino acids for secretion. The mature human II,-22 protein (i.e. hIL-22) is 146 amino acids in length and has 80.8% sequence identity with murine IL-22 (the latter being 147 amino acids in length). The amino acid sequence of hIL-22 is identified herein as SEQ ID NO. 1. Like other IL-10 family members, the IL-22 structure contains six $\alpha$-helices (referred to as helices A to F).

The derivatives for use in the invention may thus have the native amino acid sequence of hIL-22. Alternatively, they may have one or more amino acid sequence variations within the native sequence. They may additionally or alternatively include one or more amino acid sequence variations relative to (i.e. outside) the native sequence. Thus, in an embodiment, the derivative comprises a fatty acid covalently attached to hIL-22 or a variant thereof.

Expressions such as "within", "relative to", "corresponding to" and "equivalent to" are used herein to characterise the site of change and/or covalent attachment of a fatty acid in an IL-22 protein by reference to the sequence of the native protein, e.g. hIL-22. In SEQ ID NO. 1, the first amino acid residue of hIL-22 (alanine (Ala)) is assigned position 1.

Thus, a variation within the sequence of hIL-22 is a variation to any of residue numbers 1-146 in SEQ ID NO. 1. For example, a Glu substitution for the native Asp at residue 10 in hIL-22 is represented herein as "D10E". If the derivative also has a fatty acid covalently attached at position 10, it is herein referred to as attachment at residue "10E".

A variation relative to the sequence of hIL-22, however, is a variation external to residue numbers 1-146 in SEQ ID NO. 1. For example, Derivative 2 as defined herein includes an N-terminal peptide of 15 amino acids in length. The residues in the N-terminal peptide are numbered negatively, starting from the residue attached to residue 1 in hIL-22, i.e. the first residue in the N-terminal peptide that is attached to residue 1 in hIL-22 is denoted "-1". Thus, as Derivative 2 has a fatty acid covalently attached at the 7th residue in the N-terminal peptide starting from position -1 and this is Cys, the covalent attachment site for Derivative 2 is herein referred to as "-7C". Naturally, however, the numbering used in the sequence listing for Derivative 2 starts from 1, in accordance with WIPO Standard ST.26; as such, position 1 in the sequence listing for Derivative 2 is actually residue -7 as referred to herein.

Two, three, four, five or more variations may be made within the native sequence to form the derivatives used in the invention. For example, more than 10, 15, 20, 25, 50, 75, 100 or even more than 125 variations may be made in this regard. Any of residues 1-146 in the native sequence may be varied. Exemplary residues for variation are residues 1, 2, 3, 4, 5, 6, 7, 8, 9, 10, 11, 12, 13, 14, 15, 16, 17, 18, 19, 20, 21, 22, 24, 25, 26, 27, 29, 30, 32, 33, 34, 35, 36, 37, 38, 39, 40, 41, 42, 44, 45, 47, 48, 49, 50, 51, 52, 53, 54, 55, 56, 58, 59, 61, 62, 63, 64, 65, 67, 68, 69, 70, 71, 72, 73, 74, 75, 77, 78, 79, 82, 83, 84, 86, 88, 90, 91, 92, 93, 94, 95, 96, 97, 98, 99, 100, 102, 103, 104, 105, 106, 107, 108, 109, 110, 111, 112, 113, 114, 115, 116, 117, 118, 119, 120, 121, 122, 123, 124, 126, 127, 128, 129, 130, 132, 133, 134, 135, 137, 138, 139, 141, 143, 144, 145 and/or 146 in hIL-22. Variation at residues 1, 21, 35, 64, 95, 106,113 and/or 114 is particularly advantageous.

The variations within the native sequence are typically amino acid substitutions. The term "substitution", as used herein, can mean the replacement of an amino acid in the native protein with another. They may be conservative or non-conservative substitutions. Exemplary substitutions are A1C, A1G, A1H, P2C, P2H, I3C, I3H, I3V, S4H, S4N, S5H, S5T, H6C, H6R, C7G, R8G, R8K, L9S, D10E, D10S, K11C, K11G, K11V, S12C, N13C, N13G, F14S, Q15C, Q15E, Q16V, P17L, Y18F, I19Q, T20V, N21C, N21D, N21Q, R22S, F24H, M25E, M25L, L26S, A27L, E29P, A30Q, L32C, L32R, A33C, A33N, D34F, N35C, N35D, N35H, N35Q, N36Q, T37C, T37I, D38L, V39Q, R40W, L41Q, I42P, E44R, K45A, F47T, H48G, H48R, G49N, V50S, S51C, M52A, M52C, M52L, M52V, S53C, S53K, S53Y, E54D, E54F, R55Q, R55V, C56Q, L58K, M59I, Q61E, V62D, L63C, N64C, N64D, N64Q, N64W, F65G, L67Q, E69D, E69L, V70S, L71C, F72D, F72L, P73C, P73L, Q74T, R77I, F78Q, Q79E, M82Y, Q83G, E84R, V86A, F88N, A90P, A90T, R91C, R91K, R91Y, L92R, S93Y, N94C, N94Q, R95C, R95K, R95Q, L96E, S97K, T98C, T98N, T98S, C99V, H100S, E102S, G103D, D104Y, D105Y, L106C, L106E, L106Q, H107L, H107N, I108L, Q109Y, R110C, R110K, N111K, V112E, Q113C, Q113R, K114C, K114R, L115V, K116Y, D117E, T118G, V119A, K120H, K121R, L122A, G123V, G126Y, E127C, I128V, K129V, G132Y, E133Q, L134P, D135M, L137D, F138R, M139L, M139R, L141Q, N143S, A144E, C145E, I146R and/or I146V. Advantageously, the substitution may be selected from the group consisting of A1C, A1G, A1H, N21C, N21D, N21Q, N35C, N35D, N35H, N35Q, N64C, N64D, N64Q, N64W, R95C, L106C, Q113C, Q113R, K114C and K114R. Surprisingly, substitutions as employed in the invention do not adversely affect IL-22 activity.

Particular combinations of substitutions include (i) A1G, N21D, N35D and N64D; (ii) A1G, I3V, S4N, S5T, H6R, R8K, D10E, K11V, T20V, H48R, M52A, S53K, E54D, R55Q, E69D, F72L, A90T, R91K, R95Q, T98S, E102S, L106Q, H107N, R110K, Q113R, K114R, D117E and I146V; (iii) A1G, I3V, S4N, S5T, H6R, R8K, D10E, K11V, T20V, H48R, M52A, S53K, E54D, R55Q, E69D, F72L, A90T, R91K, R95Q, T98S, E102S, L106Q, H107N, R110K, Q113R, K114R, D117E and I146V; (iv) A1G, N35Q and N64Q; (v) A1G and N64C; (vi) A1G and Q113C; (vii) A1G and K114C; (viii) A1G and M25L; (ix) A1G and M52L; (x) A1G and M139L; (xi) A1G and N36Q; (xii) A1G and D117E; (xiii) A1G and N21Q; (xiv) A1G and N35Q; (xv) A1G and N64Q; (xvi) A1G, N21Q and N35Q; (xvii) A1G, N21Q and N64Q; (xviii) A1G, N21Q, N35Q and N64Q; (xix) A1G and K11C; (xx) A1G and N13C; (xxi) N35Q and N64Q; (xxii) A1C, N35Q and N64Q; (xxiii) H6C, N35Q and N64Q; (xxiv) I3C, N35Q and N64Q; (xxv) P2C, N35Q and N64Q; (xxvi) L32C, N35Q and N64Q; (xxvii) N35Q, M52C and N64Q; (xxviii) N13C, N35Q and N64Q; (xxix) N21C, N35Q and N64Q; (xxx) N35Q, N64Q and N94C; (xxxi) N35Q, N64Q and P73C; (xxxii) N35Q, N64Q and Q113C; (xxxiii) N35Q, N64Q and R91C; (xxxiv) N35Q, N64Q and R95C; (xxxv) N35Q, N64Q and L106C; (xxxvi) N35Q, N64Q and R110C; (xxxvii) S12C, N35Q and N64Q; (xxxviii) N35Q, S51C and N64Q; (xxxix) N35Q, S53C and N64Q; (xxxx) N35Q, T37C and N64Q; (xxxxi) N35Q, N64Q and T98C; (xxxxii) Q15C, N35Q and N64Q; (xxxxiii) N35C and N64Q; (xxxxiv) H6C, N35Q and N64Q; (xxxxv) A33C, N35Q and N64Q; and (xxxxvi) A1H, P2H, I3H, S4H, S5H, C7G, R8G, L9S, D10S, K11G, N13G, F14S, Q15E, Q16V, P17L, 18F, Y19Q, N21Q, R22S, F24H, M25E, L26S, A27L, E29P, A30Q, L32R, A33N, D34F, N35H, T37I, D38L, V39Q, R40W, L41Q, I42P, E44R, K45A, F47T, H48G, G49N, V50S, M52V, S53Y, E54F, R55V, C56Q, L58K, M59I, Q61E, V62D, L63C, N64W, F65G, L67Q, E69L, V70S, L71C, F72D, P73L, Q74T, R77I, F78Q, Q79E, M82Y, Q83G, E84R, V86A, F88N, A90P, R91Y, L92R, S93Y, N94Q, R95K, L96E, S97K, T98N, C99V, H100S, G103D, D104Y, D105Y, L106E, H107L, I108L, Q109Y, R111K, V112E, L115V, K116Y, D117E, T118G, V119A, K120H, K121R, L122A, G123V, G126Y, E127C, I128V, K129V, G132Y, E133Q, L134P, D135M, L137D, F138R, M139R, L141Q, N143S, A144E, C145E and I146R. Any and all combinations of substitutions are envisaged and form part of the invention.

A derivative for use in the first or second aspect may typically comprise an amino acid substitution whereby Cys is substituted for a native residue, optionally in any of the positions identified above, such as position 1, 2, 3, 6, 11, 12, 13, 15, 21, 32, 33, 35, 37, 51, 52, 53, 63, 64, 71, 73, 91, 94, 95, 98, 106, 110, 113, 114 and/or 127. Advantageously, the IL-22 protein included in a derivative for use in the first or second aspect comprises a Cys residue at position 1 of hIL-22. An A1C substitution combined with substitutions in two glycosylation sites at positions 35 and 64 is particularly advantageous, as it leads to faster uptake without adversely affecting potency or half-life (see Derivatives 6 and 10 in Examples 1 and 2 of WO 2021/089875; incorporated herein by reference). Alternatively, the IL-22 protein included in a derivative for use in the first or second aspect comprises the substitution R95C (as per Derivative 14) or L106C (as per Derivative 13). Such a substitution combined with substitutions in the two glycosylation sites at positions 35 and 64 (as per Derivatives 11 and 12) is particularly advantageous, as it leads to faster uptake without adversely affecting potency or half-life (see Derivatives 1 and 2 in Example 1 of WO 2022/238510; incorporated herein by reference). In one advantageous embodiment, a derivative for use in the first or second aspect comprises the substitutions, N35Q, N64Q and R95C. In another advantageous embodiment, a derivative for use in the first or second aspect comprises the substitutions, N35Q, N64Q and L106C. However, in other embodiments, it may be advantageous that a derivative for use in the first or second aspect comprises said R95C or said L106C without additional substitutions or variations within hIL-22 (SEQ ID NO. 1).

Alternatively, or in addition, the variations within the native sequence may be amino acid insertions. Up to five, 10, 15, 20, 25, 30, 35, 40, 45 or even up to 50 amino acids may be inserted within the native sequence. Trimers, pentamers, septamers, octamers, nonamers and 44-mers are particularly advantageous in this regard. Exemplary sequences are shown in Table 1. Insertions can be made at any location in the native sequence, but those in helix A (for example, at residue 30), loop CD (for example, at residue 75), helix D (for example, at residue 85) and/or helix F (for example, at residue 124) are preferred.

*Table 1: Sequence of exemplary amino acid insertions*

| n-mer | Exemplary amino acid sequence | SEQ ID NO. |
|---|---|---|
| Trimer | E-T-S | n/a |
| Pentamer | R-V-Q-F-Q or C-V-E-I-P | 2, 3 |
| Septamer | G-S-G-S-G-S-C | 4 |
| Octamer | I-E-A-L-T-P-H-S or Y-G-Q-R-Q-W-K-N | 5, 6 |
| Nonamer | V-F-I-I-N-N-S-L-E | 7 |

(continued)

| n-mer | Exemplary amino acid sequence | SEQ ID NO. |
|---|---|---|
| 44-mer | R-A-A-S-A-G-S-Y-S-E-W-S-M-T-P-R-F-T-P-W-W-E-T-K-I-D-P-P-V-M-N-I-T-Q-V-N-G-S-L-L-V-I-L-H | 8 |

The one, two, three, four, five or more variations within the native sequence may be independently selected from the group consisting of substitutions and insertions.

The variations within the native sequence may also or alternatively comprise one or more amino acid deletions within SEQ ID NO. 1. The peptide may thus comprise up to five amino acid deletions. No more than three or two amino acid deletions are preferred. Said deletions may be present in separate (i.e. non-consecutive) positions, e.g., within SEQ ID NO. 1. The variations may also or alternatively be a deletion of two, three, four or five consecutive amino acids within SEQ ID NO. 1, meaning that a series of up to five neighbouring amino acids may be deleted.

Sequence variations relative to the amino acid sequence of hIL-22, if present, typically include an extension, such as the addition of a peptide at the N-terminal end. The peptide may consist of up to five, 10, 15, 20, 25, 30, 35, 40, 45 or even up to 50 amino acids. Monomers, trimers, octamers, 13-mers, 15-mers, 16-mers, 21-mers, 28-mers are particularly advantageous in this regard. Exemplary sequences are shown in Table 2. Suitably, the IL-22 protein included in a derivative for use in the first or second aspect comprises an N-terminal G-P-G. In a particularly preferred example, the derivative for use in the first or second aspect comprises both a Cys residue at position 1 of hIL-22 (SEQ ID NO. 1) and an N-terminal G-P-G. This has been found to create a derivative with a very good half-life and potency (see Derivatives 1, 3 and 5 in Examples 1 and 2 of WO 2021/089875; incorporated herein by reference). In an embodiment, the IL-22 protein included in a derivative for use in the first or second aspect does not comprise an N-terminal G-P-G.

*Table 2: Sequence of exemplary N-terminal peptides*

| n-mer | Exemplary amino acid sequence | SEQ ID NO. |
|---|---|---|
| Monomer | C, G or M | n/a |
| Trimer | G-P-G | n/a |
| Pentamer | A-E-P-E-E | 9 |
| Heptamer | G-P-A-E-P-E-E | 10 |
| Octamer | G-P-A-C-E-P-E-E | 11 |
| 12-mer | G-S-G-S-G-S-G-S-G-S-G-S | 12 |
| 13-mer | G-G-S-S-G-S-G-S-E-V-L-F-Q | 13 |
| 14-mer | G-P-G-S-G-S-G-S-G-S-G-S-G-S | 14 |
| 14-mer | G-G-S-S-G-S-G-S-E-V-L-F-Q-G | 15 |
| 15-mer | G-P-G-S-G-S-G-S-C-G-S-G-S-G-S | 16 |
| 16-mer | G-G-S-S-G-S-G-S-E-V-L-F-Q-G-P-G | 17 |
| 20-mer | G-G-S-S-G-S-G-S-E-V-L-F-Q-G-P-A-E-P-E-E | 18 |
| 21-mer | G-G-S-S-G-S-G-S-E-V-L-F-Q-G-P-A-C-E-P-E-E | 19 |
| 27-mer | G-G-S-S-G-S-G-S-E-V-L-F-Q-G-P-G-S-G-S-G-S-G-S-G-S-G-S | 20 |
| 28-mer | G-G-S-S-G-S-G-S-E-V-L-F-Q-G-P-G-S-G-S-G-S-C-G-S-G-S-G-S | 21 |

Sequence variations relative to the amino acid sequence of hIL-22, if present, may include the addition of a peptide at the C-terminal end. The peptide may consist of up to five, 10, 15, 20, 25, 30, 35, 40, 45 or even up to 50 amino acids. Exemplary C-terminal peptide sequences include those shown in Table 2 (for N-terminal peptides). A septamer is particularly advantageous in this regard, optionally having the amino acid sequence, G-S-G-S-G-S-C (SEQ ID NO. 22).

The derivatives for use in the invention may include both an N-terminal and a C-terminal peptide in addition to the native or variant hIL-22 amino acid sequence as herein described. Any combination of the N- and C-terminal peptides described herein is envisaged and expressly included in the invention.

It will be appreciated that the invention extends to any derivative of IL-22, which comprises a fatty acid covalently attached to hIL-22 or a variant thereof. The "variant" can be a protein having at least 10% sequence identity with hIL-22. In an embodiment, the variant has at least 20%, or even at least 30%, sequence identity with hIL-22. The variant may have "substantially the amino acid sequence" of hIL-22, which can mean a sequence that has at least 40% sequence identity with the amino acid sequence of hIL-22. Accordingly, in an embodiment, a derivative for use in the first or second aspect has at least 50%, at least 60%, at least 70%, at least 80%, at least 90%, at least 95% or 100% amino acid sequence identity with hIL-22. Exemplary IL-22 protein variants are set forth in SEQ ID NOs. 23-28.

The skilled technician will appreciate how to calculate the percentage identity between two amino acid sequences. An alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local *versus* global alignment, the pair-score matrix used (for example, BLOSUM62, PAM250, Gonnet etc.) and gap-penalty, for example, functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length-dependent. Therefore, the shorter a pair of sequences is, the higher the sequence identity one may expect to occur by chance.

Hence, it will be appreciated that the accurate alignment of amino acid sequences is a complex process. The popular multiple alignment program, ClustalW, is a preferred way for generating multiple alignments of proteins in accordance with the invention. Suitable parameters for ClustalW may be as follows: For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)*100.

Alternative methods for identifying similar sequences will be known to those skilled in the art.

Suitably, a derivative for use in the first or second aspect comprises 200 amino acids or less. For example, the derivative comprises less than 190, less than 180, less than 170, less than 160 or even less than 150 amino acids. Suitably, the derivative will comprise at least 146 amino acids, however, this being the number of amino acids in hIL-22. It may comprise at least 150 amino acids, at least 160 amino acids, at least 170 amino acids or even at least 180 amino acids. The derivatives for use in the invention can comprise proteins of any length within the above ranges, but they will typically be 146-180 amino acids in length.

The derivatives for use in the invention, whether having the native or a variant amino acid sequence, include a fatty acid covalently attached to the IL-22 protein. The fatty acid is typically covalently attached to the IL-22 protein by a linker. The fatty acid and linker are suitably connected to each other via an amide bond, and the linker is covalently attached to the IL-22 protein. The fatty acid and linker may thus be present as a side chain on the IL-22 protein. It was surprising to the inventors at the time that a covalently attached fatty acid does not adversely affect IL-22 activity. It was particularly surprising that fatty acid attachment is associated with additional advantages, such as prolongation of half-life.

The fatty acid may be any suitable fatty acid. In particular, the fatty acid may be of Formula I:

$$HOOC\text{-}(CH_2)_x\text{-}CO\text{-}*,$$

wherein x is an integer in the range of 10-18, optionally 12-18, 14-16 or 16-18, and * designates a point of attachment to the IL-22 protein or linker. It may be a fatty diacid, such as a C12, C14, C16, C18 or C20 diacid. Advantageously, the fatty acid is a C16 or C18 diacid, and most advantageously it is a C18 diacid.

For example, $-(CH_2)_x-$ in Formula I may be a straight alkylene in which x is 10. This fatty acid may be conveniently referred to as C12 diacid, i.e. a fatty di-carboxylic acid with 12 carbon atoms. Alternatively, $-(CH_2)_x-$ in Formula I may be a straight alkylene in which x is 12. This fatty acid may be conveniently referred to as C14 diacid, i.e. a fatty di-carboxylic acid with 14 carbon atoms. In a similar fashion, $-(CH_2)_x-$ in Formula I may be a straight alkylene in which x is 14 (C16 diacid), 16 (C18 diacid) or 18 (C20 diacid). Suitably, a derivative for use in the first or second aspect includes a C14, C16, C18 or C20 diacid; more suitably, a C16 or C18 diacid, and even more suitably a C18 diacid.

The diacid may be capable of forming non-covalent associations with albumin, thereby promoting circulation of the derivative in the blood stream. The shorter diacids (e.g. C16 diacid) have lower albumin affinity and thus a shorter half-life than the longer diacids (e.g. C18 diacid). However, they are still long acting derivatives with an expected half-life in man of over one day.

Fatty acid attachment will, in itself, also stabilise the IL-22 protein against proteolytic degradation. The resulting half-life is typically similar to that of IL-22-Fc fusions (i.e. greatly improved compared to hIL-22).

The derivatives for use in the first or second aspect may comprise particular combinations of a fatty acid and IL-22 protein. For example, a C14, C16, C18 or C20 diacid may be attached to an IL-22 protein comprising a Cys residue at position 1 of hIL-22 and/or an N-terminal G-P-G. In one example, a derivative for use in the first or second aspect comprises a C18 diacid and the IL-22 protein comprises both a Cys residue at position 1 of hEL-22 and an N-terminal G-P-G. As another example, a derivative for use in the first or second aspect comprises a C18 diacid and the IL-22 protein comprises a Cys residue substituted at position 95 or 106 of hIL-22. Such a derivative may further comprise a Gln residue substituted at positions 35 and 64 of hIL-22 (such as in Derivatives 11 and 12). In these examples, the IL-22 protein may additionally comprise an N- or C- terminal pentamer having the sequence, A-E-P-E-E (SEQ ID NO. 9).

As above, the fatty acid is suitably connected to a linker, which is attached to the IL-22 protein. The linker may comprise several linker elements, including one or more amino acids such as one or more Glu and/or Lys residues. The linker may include an oxyethylene glycine unit or multiple linked oxyethylene glycine units, optionally 2-5 such units, advantageously 2 units. One or more OEG residues, $C_2DA$ and/or Ac groups may alternatively or additionally be included. The linker may comprise a Cys-reactive unit. A "Cys-reactive unit", as used herein, can mean a functional unit that is able to react with the sulphur atom of a Cys to create a carbon-sulphur covalent bond. The Cys-reactive unit can have any of several forms, but suitably includes a carbon atom attached to a leaving group, which leaving group becomes displaced by the sulphur atom of the Cys during formation of the carbon-sulphur bond. The leaving group may be a halogen, optionally a bromine atom. This bromide leaving group can be alpha to an Ac functional group; advantageously it is a bromo-Ac functional group. The leaving group may alternatively be a functionalised hydroxyl group of the form mesylate or tosylate, or an unfunctionalised hydroxyl group. Further, the leaving group can be a maleimide or other functional group. Exemplary linkers include γGlu-OEG-OEG-$C_2DA$-Ac, yGlu-yGlu-yGlu-γGlu-OEG-OEG-εLys-αAc and γGlu-OEG-OEG-εLys-αAc, but any suitable linker may be employed.

A variant comprising a fatty diacid covalently attached to a linker selected from yGlu-OEG-OEG-$C_2DA$-$A_C$ and γGlu-γGlu-γGlu-γGlu-OEG-OEG-εLys-αAc may be preferred in some embodiments. A variant comprising a C14, C16, C18 or C20 fatty diacid covalently attached to a linker selected from γGlu-OEG-OEG-$C_2DA$-Ac and yGlu-yGlu-yGlu-yGlu-OEG-OEG-εLys-αAc may be preferred in some embodiments. A variant comprising a C14 fatty diacid covalently attached to the linker, γGlu-γGlu-γGlu-γGlu-OEG-OEG-εLys-αAc, may be preferred in some embodiments. A variant comprising a C16, C18 or C20 fatty diacid covalently attached to the linker, γGlu-γGlu-γGlu-γGlu-OEG-OEG-εLys-αAc, may be preferred in some embodiments.

The linker may be a Cys-reactive linker attached to a Cys residue within SEQ ID NO. 1. The linker may be a Cys-reactive linker attached to a Cys residue in an extension of the C-terminal or N-terminal relative to SEQ ID NO. 1.

The fatty acid, or linker, may be attached to any amino acid residue in the IL-22 protein. Exemplary in this regard are residues -7, -5, 1, 6, 33, 95, 106, 113, 114 and 153 in or relative to the hIl-22 amino acid sequence. The native residue is typically substituted with Cys or Lys to enable attachment of the fatty acid or linker. Alternatively the fatty acid or linker can be attached at a native Cys or Lys residue. Suitably, the fatty acid or linker is attached to a Cys residue substituted at position 1, 6, 33, 95, 106, 113 or 114 of hEL-22 or to a Cys residue at position -5, -7 or 153 relative to hIL-22. In particular, the fatty acid or linker may be attached to a Cys residue substituted at position 1 of hEL-22. Alternatively, the fatty acid or linker may be attached to a Cys residue substituted at position 95 or 106 of hIL-22.

The attachment of the fatty acid or linker to the IL-22 protein is a covalent attachment. For example, a Cys-reactive fatty acid or linker may be used to attach the fatty acid or linker to a Cys residue in the IL-22 protein. The fatty acid or linker may be covalently attached to the sulphur atom of the Cys residue via a thioether bond. Alternatively, a Lys-reactive fatty acid or linker may be used to attach the fatty acid or linker to a Lys residue in the IL-22 protein. The fatty acid or linker may alternatively be covalently attached to the free amine (-$NH_2$) group in the N-terminus of the IL-22 protein (irrespective of the amino acid in position 1). Attachment can proceed as with Cys attachment, albeit with sub-stoichiometric amounts of fatty acid or linker containing a suitable N-reactive species. The fatty acid or linker may be presented in the form of an aldehyde (the N-reactive species) and be covalently attached to the free amine employing a classically known reductive amination.

A derivative for use in the first or second aspect thus suitably comprises a C14, C16, C18 or C20 diacid attached by a linker to a variant of hIL-22, wherein the variant comprises an N-terminal G-P-G and a Cys residue at position 1 of hIL-22 and the linker is optionally attached to the Cys residue. Alternatively, a derivative for use in the first or second aspect suitably comprises a C14, C16, C18 or C20 diacid attached by a linker to a variant of hIL-22, wherein the variant comprises a Cys residue at position 95 or 106 of hIL-22 and the linker is optionally attached to the Cys residue. Optionally a Gln residue is substituted at positions 35 and/or 64.

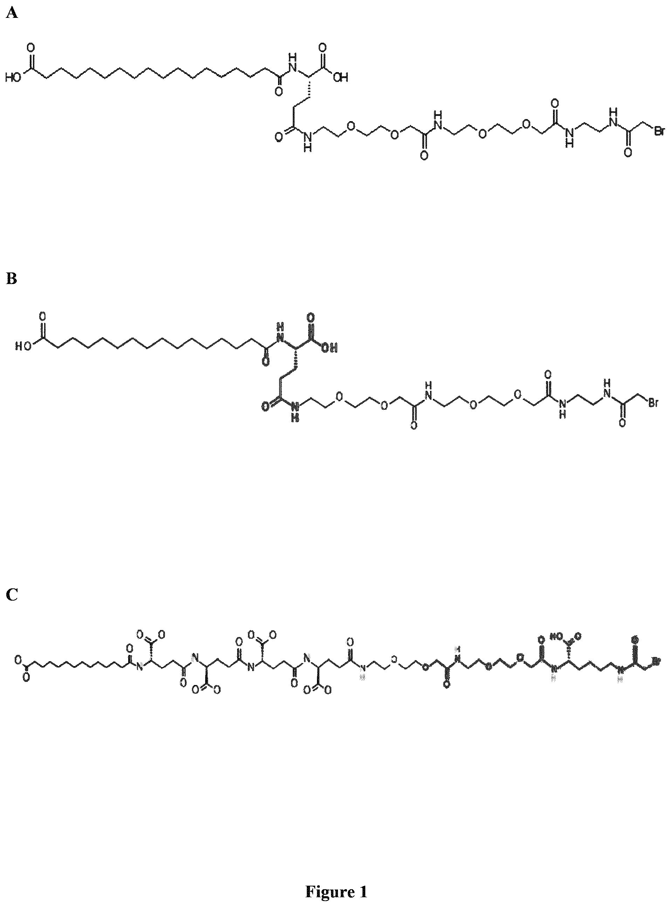

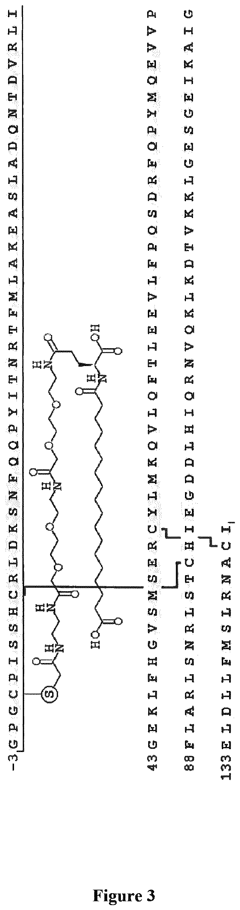

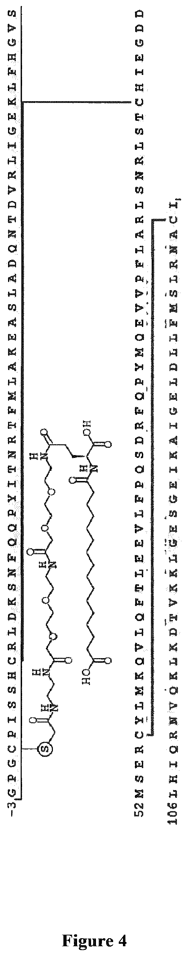

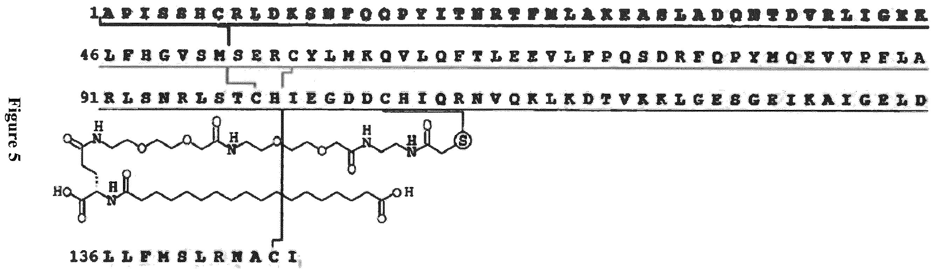

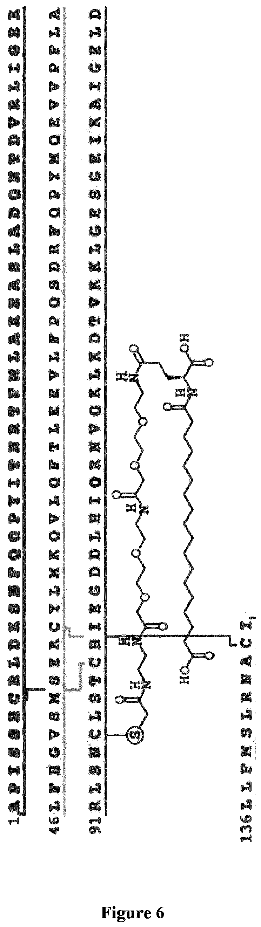

Exemplary derivatives for use in the first or second aspect comprise an IL-22 protein as set forth in any of SEQ ID NOs. 23-32. Particularly advantageous derivatives are shown in Table 3 and illustrated in Figures 1-6. Derivatives 1 and 6 are exemplified herein.

*Table 3: Exemplary derivatives of IL-22*

| ID | Sequence variations | SEQ ID NO. | Covalent attachment site | Linker | Fatty acid |
|---|---|---|---|---|---|
| Derivative 1 | A1C substitution & G-P-G N-terminal peptide | 23 | 1C | γGlu-OEG-OEG-C$_2$DA-Ac | C18 diacid |
| Derivative 2 | G-P-G-S-G-S-G-S-C-G-S-G-S-G-S N-terminal peptide | 24 | -7C | γGlu-OEG-OEG-C$_2$DA-Ac | C18 diacid |
| Derivative 3 | A1C substitution & G-P-G N-terminal peptide | 23 | 1C | γGlu-OEG-OEG-C$_2$DA-Ac | C16 diacid |
| Derivative 4 | G-P-G-S-G-S-G-S-C-G-S-G-S-G-S N-terminal peptide | 24 | -7C | γGlu-OEG-OEG-C$_2$DA-Ac | C16 diacid |
| Derivative 5 | A1C substitution & G-P-G N-terminal peptide | 23 | 1C | γGlu-γGlu-γGlu-γGlu-OEG-OEG-εLys-αAc | C14 diacid |
| Derivative 6 | A1C, N35Q, N64Q substitutions & G-P-G N-terminal peptide | 25 | 1C | γGlu-OEG-OEG-C$_2$DA-Ac | C18 diacid |
| Derivative 7 | A1C, N35Q, N64Q substitutions | 26 | 1C | γGlu-OEG-OEG-C$_2$DA-γ Ac | C18 diacid |
| Derivative 8 | H6C, N35Q, N64Q substitutions & G N-terminal peptide | 27 | 6C | γGlu-OEG-OEG-C$_2$DA-Ac | C18 diacid |
| Derivative 9 | A33C, N35Q, N64Q substitutions | 28 | 33C | γGlu-OEG-OEG-C$_2$DA-Ac | C18 diacid |
| Derivative 10 | A1C, N35Q, N64Q substitutions & G-P-G N-terminal peptide | 25 | 1C | γGlu-OEG-OEG-C$_2$DA-Ac | C16 diacid |
| Derivative 11 | N35Q, N64Q, L106C substitutions | 29 | 106C | γGlu-OEG-OEG-C$_2$DA-Ac | C18 diacid |
| Derivative 12 | N35Q, N64Q, R95C substitutions | 30 | 95C | γGlu-OEG-OEG-C$_2$DA-Ac | C18 diacid |
| Derivative 13 | L106C substitution | 31 | 106C | γGlu-OEG-OEG-C$_2$DA-Ac | C18 diacid |
| Derivative 14 | R95C substitution | 32 | 95C | γGlu-OEG-OEG-C$_2$DA-Ac | C18 diacid |

Figure 1A illustrates a C18 diacid connected to a linker comprising a Cys-reactive unit. This is the fatty acid and linker (side chain) used in Derivatives 1, 2, 6-9 and 11-14. Figure 1B illustrates a C16 diacid connected to a linker comprising a Cys-reactive unit. This is the fatty acid and linker (side chain) used in Derivatives 3, 4 and 10. Figure 1C illustrates a C14 diacid connected to a linker comprising a Cys-reactive unit. This is the fatty acid and linker (side chain) used in Derivative 5.

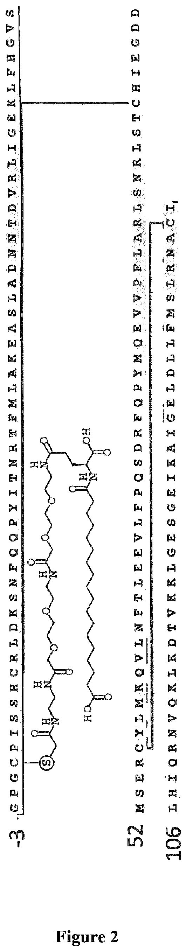

Derivatives 1, 6 and 10-14 are illustrated in Figures 2-6, respectively.

The derivatives for use in the invention may exist in different stereoisomeric forms and the invention relates to all of these.

**II. Process for Preparing Derivatives of IL-22**

A derivative for use in the first or second aspect may be prepared by a process comprising covalently attaching a fatty acid to an IL-22 protein.

The process may be used to produce any of the different derivatives of IL-22 described or envisaged herein, but it is particularly advantageous when a fatty acid is covalently attached to a variant IL-22 protein. Thus, the IL-22 protein employed in the process may be a substituted form of hIL-22, optionally substituted at position 1, 21, 35, 64, 95, 106, 113 and/or 114. Exemplary substitutions include A1C, A1G, A1H, N21C, N21D, N21Q, N35C, N35D, N35H, N35Q, N64C, N64D, N64Q, N64W, R95C, L106C, Q113C, Q113R, K114C and/or K114R. Preferably, the IL-22 protein is substituted with a Cys residue at position 1. Alternatively, the IL-22 protein is substituted with a Cys residue at position 95 or 106, optionally with a Gln residue at position 35 and/or 64 too.

The fatty acid can be obtained by any means known in the art, including recombinant means. Suitable fatty acids are commercially available or readily derived from available starting materials using standard chemical synthesis.

The IL-22 protein can be obtained by any means known in the art, including recombinant means. The production of recombinant hIL-22 has been previously described and is well-known in the art. Desired variant IL-22 proteins can be produced in a similar manner. An experienced investigator in the field would be readily able to identify suitable nucleic acid sequences that encode the desired variant IL-22 proteins. The skilled person would hence be readily able to execute this part of the invention, based upon the existing knowledge in the art. Suitably, the IL-22 proteins are produced in mammalian systems, such as in Chinese hamster ovary (CHO) cells, using standard techniques. A polyhistidine tag (His-tag) may be employed to aid affinity purification of the recombinant proteins.

In this regard, IL-22 proteins for use in the invention can be prepared using a post expression cleavable His-tag - an N- or C-terminal addition of less than 10, preferably six, histidine residues that can be purified by affinity to a nickel column. The His-tag is linked to the N- or C-terminal of the protein via a linker that can be digested by a known protease to leave the free IL-22 protein. The cleavable His-tag can have the amino acid sequence, HHHHHHGGSSGSGSEVLFQ (SEQ ID NO. 33), and the protease-cleavable linker can be a tobacco etch virus (TEV) linker, whose consensus sequence for the native cut sites is ENLYFQ\S (SEQ ID NO. 34), where '\' denotes the cleaved peptide bond or a human rhinovirus-14 3C (HRV14-3C) protease cleavable linker with EVLFQ consensus cleavage site.

Cleavage may be achieved by incubating approximately 10 μg protease with 2.5 μg protein and 10 mM 2-mercaptoethanol at room temperature for 4h.

A representative process for protein preparation is provided as follows. The process involves preparing a plasmid DNA that encodes the desired amino acid sequence of the IL-22 protein. This plasmid can be transiently transfected into a cell line, for example CHO-K1, which is allowed to grow in a relevant medium before growth is increased by the addition of a known enhancer. The secreted IL-22 protein can then be harvested through known methods of centrifugation and sterile filtration before the protein is purified on a nickel column. Following concentration and buffer exchange the His-tag is removed using a HRV14-3C protease before alkylation with a fatty acid (described further below) and final purification and buffer exchange. Analysis of the final product using sodium dodecyl-sulfate polyacrylamide gel electrophoresis (SDS-PAGE), size exclusion chromatography or liquid chromatography with tandem mass spectrometry (LC-MS-MS) with, or without, deglycosylation can be used to ensure the quality of the final product.

The fatty acid can be covalently attached to the IL-22 protein either directly or using a linker as described for the first or second aspect. The linker can be obtained by any means known in the art. A representative method for preparing the fatty acid and linker, if employed, is as follows (exemplified by the C16 diacid used in Derivative 10, but any derivative could be made using a similar method).

A solution of N-(benzyloxycarbonyloxy) succinimide (100 g, 401 mmol) in dichloromethane (500 ml) is added to a solution of ethylene diamine (189 ml, 2.81 mol) in dichloromethane (750 ml). After 30 minutes the suspension is filtered, washed and concentrated *in vacuo.* The residue is diluted with toluene (750 ml), washed and extracted with dichloromethane (4 × 200 ml), dried over anhydrous sodium sulphate, filtered, concentrated *in vacuo* and diluted with hexanes (200 ml). A 4 M solution of hydrogen chloride in ether (100 ml, 400 mmol) is added to the solution, the resulting suspension concentrated *in vacuo* and diluted with hexanes (1 l). The precipitated solid is filtered, washed with hexanes and dried *in vacuo* to give (2-aminoethyl) carbamic acid benzyl ester hydrochloride as a white powder.

2-Chlorotrityl resin 100-200 is loaded with {2-[2-(9H-fluoren-9-ylmethoxycarbonylamino)-ethoxy]-ethoxy}-acetic acid (Fmoc-Ado-OH, 17.5 g, 45.4 mmol) The Fmoc group is removed and a solution of 0-6-chloro-benzotriazol-1-yl)-N,N,N',N'-tetramethyluronium tetrafluoroborate (TCTU, 24.2 g, 68.1 mmol) and N,N-diisopropylethylamine (21.4 ml, 123 mmol) in N,N dimethylformamide (140 ml) is added to the resin and the mixture shaken for one hour. The resin is filtered and washed. The Fmoc group is removed by treatment with 20% piperidine as before. The resin is washed as before.

A solution of (S)-2-(fluoren-9-ylmethoxycarbonylamino)-pentanedioic acid 1-tert-butyl ester (Fmoc-Glu-OtBu, 29.0 g, 68.1 mmol), TCTU (24.2 g, 68.1 mmol) and N,N-diisopropylethylamine (21.4 ml, 123 mmol) in N,N dimethylformamide (140 ml) is added to the resin and the mixture shaken for one hour. The resin is filtered and washed as before.

The Fmoc group is removed by treatment with 20% piperidine as before. The resin is washed as before.

A solution of 16-tert-butoxy)-16-oxohexadecanoic acid (23.3 g, 68.1 mmol), TCTU (24.2 g, 68.1 mmol) and N,N diisopropylethylamine (21.4 ml, 123 mmol) in N,N-dimethylformamide/dichloromethane mixture (4:1, 200 ml) is added to the resin. The resin is shaken for one hour, filtered and washed with N,N-dimethylformamide (3 × 250 ml), dichloromethane (2 × 250 ml), methanol (2 × 250 ml) and dichloromethane (6 × 250 ml). The product is cleaved from the resin by treatment with 2,2,2-trifluoroethanol (250 ml) for 18 hours. The resin is filtered off and washed with dichloromethane (2 × 250 ml), 2-propanol/dichloromethane mixture (1:1, 2 × 250 ml), 2-propanol (250 ml) and dichloromethane (3 × 250 ml).

The solutions are combined, the solvent is evaporated and crude product purified by flash column chromatography. Pure (S)-22-(tert-butoxycarbonyl)-41,41-dimethyl-10,19,24,39-tetraoxo-3,6,12,15,40-pentaoxa-9,18,23-triazadotetracontanoic acid is dried *in vacuo* and obtained as a pale yellow thick yellow oil.

2-(7-Aza-1H-benzotriazole-1-yl)-1,1,3,3-tetramethyluronium hexafluorophosphate (HATU, 11.4 g, 30.1 mmol) and triethylamine (8.77 ml, 62.9 mmol) are subsequently added to a solution of (S)-22-(tert-butoxycarbonyl)-41,41-dimethyl-10,19,24,39-tetraoxo-3,6,12,15,40-pentaoxa-9,18,23-triazadotetracontanoic acid (22.4 g, 27.4 mmol) in dry dichloromethane (110 ml). Triethylamine (72 ml, 41.0 mmol) is added to a suspension of (2-amino-ethyl)-carbamic acid benzyl ester hydrochloride (6.94 g, 30.1 mmol) in dry dichloromethane (165 ml) and the resulting mixture is added to the above solution. The mixture is stirred at room temperature overnight and then evaporated to dryness. The residue is re-dissolved and washed; dried over anhydrous sodium sulphate and evaporated column chromatography (Silicagel 60, 0.040-0.060 mm; eluent: dichloromethane/methanol 95:5) to afford 15-[(S)3-(2-{2-[(2-{2-[(2-benzyloxycarbonylamino-ethylcarbamoyl)-methoxy]-ethoxy}ethyl-carbamoyl)methoxy]ethoxy)-ethylcarbamoyl)-1-tert-butoxycarbonylpropylcarbamoyl]-pentadecanoic acid tert-butyl ester as a pale yellow thick oil.

Palladium on carbon (10%, 1.27 g, 1.20 mmol) is added to a solution of the above compound (23.8 g, 24.0 mmol) in methanol (350 ml) and the resulting mixture hydrogenated at normal pressure for four hours. The catalyst is filtered off and the filtrate evaporated to dryness. The residue is evaporated several times from dichloromethane in order to remove residues of methanol and dried *in vacuo* to yield tert-butyl(S)-1-amino-25-tert-butoxycarbonyl)-4,13,22,27-tetraoxo-6,9,15,18-tetraoxa-3,12,21,26-tetraazadotetracontan-42-oate as a thick colourless oil.

N,N-Diisopropylethylamine (4.98 ml, 28.6 mmol) is added to a solution of the above amine (20.5 g, 23.8 mmol) in dry dichloromethane (290 ml) at - 30° C. under argon. Bromoacetyl bromide (2.48 ml, 28.6 mmol) is added dropwise and the resulting solution is stirred at -30° C for an additional three hours. The cooling bath is removed, the mixture is stirred at room temperature for one hour, and the solvent is removed *in vacuo.* The residue is re-dissolved in ethyl acetate (450 ml) and washed with 5% aqueous solution of citric acid (300 ml). The phases are separated within one hour. The organic layer is left to separate overnight to give three phases. The clear aqueous layer is removed and the residual two phases shaken with a saturated aqueous solution of potassium bromide (100 ml). The phases are left to separate overnight, the aqueous phase removed and the organic phase dried over anhydrous sodium sulphate. The solvent is removed *in vacuo* and the residue purified by flash column chromatography: dichloromethane/methanol 95:5) to afford tertbutyl(S)-1-bromo-28-tert-butoxycarbonyl)-2,7,16,25,30-pentaoxo-9,12,18,21-tetraoxa-3,6,15,24,29-pentaazapenta- tetracontan-45-oate as a colourless solid.

The above compound (19.5 g, 19.8 mmol) is dissolved in trifluoroacetic acid (120 ml) and the resulting solution is stirred at room temperature for 1.5 hours. Trifluoroacetic acid is removed *in vacuo* and the residue is evaporated from dichloromethane (6 × 200 ml). Diethyl ether (200 ml) is added to the oily residue and the mixture stirred overnight to give a suspension. The solid product is filtered, washed with diethyl ether and hexanes and dried *in vacuo* to afford the desired product 15-{(S)-1-carboxy3-[2-(2-{[2-(2-{[2-(2-bromoacetylamino)ethylcarbamoyl]methoxy}-ethoxyethyl-carbamoyl]methoxy}ethoxyl-ethylcarbamoyl]propylcarbamoyl}pentadecanoic acid as a white powder.

Covalent attachment of the fatty acid or linker to the IL-22 protein may be carried out using standard procedures in the art. The linker, if employed, thus enables covalent attachment of the IL-22 protein to the fatty acid. By way of a non-limiting example, a Cys-reactive fatty acid or linker may be reacted with the sulphur atom of a Cys residue in the IL-22 protein, so forming a thioether bond. Suitable conditions for the covalent attachment step may be exemplified as follows: Tris in water is added to IL-22 protein (70 mg) in Tris and NaCl-buffer (1.35 mg/ml), to adjust to pH 8. Bis(p-sulfonatophenyl)- phenylphosphine dihydrate dipotassium (BSPP) salt (12 mg), dissolved in water, is added and stirred gently for four hours at room temperature. 15-{(S)-1-Carboxy-3-[2-(2-{[2-(2-{[2-(2-bromoacetylamino)-ethylcarbamoyl]ethoxy}ethoxy)ethylcarbamoyl]methoxy}ethoxy)ethylcarbamoyl]propylcarbamoyl}pentadecanoic acid (19 mg, 0.022 mmol) in ethanol (0.5 ml) is added and the mixture stirred gently overnight. MiliQ water (150 ml) is added to lower the conductivity to 2.5 mS/cm. The mixture is then purified using anion exchange on a MonoQ 10/100 GL column using binding buffer (20 mM Tris, pH 8.0), elution buffer (20 mM Tris, 500 mM NaCl, pH 8.0), flow 6 ml and a gradient of 0-80% elution buffer over 60 column volumes.

The derivatives for use in the invention may be purified using any suitable procedure known in the art, such as chromatography, electrophoresis, differential solubility or extraction.

### III. Therapeutic Efficacy of Derivatives of IL-22

As aforementioned, the inventors were surprised at the time to find that fatty acids could be covalently attached to an IL-22 protein whilst maintaining biological activity. It was particularly surprising that such a minimal modification to IL-22 could result in high potency (close to hIL-22) combined with a very long circulatory half-life. This particular combination of properties may be highly desirable.

The potency of the derivatives may be determined in an *in vitro* assay with whole cells expressing human IL-22 receptors. For example, the response of the human IL-22 receptors may be measured using baby hamster kidney (BHK) cells overexpressing IL-22R1 (also known as IL-22 receptor $\alpha$ or IL22RA), IL-10R2 (also known as IL-10 receptor $\beta$ or IL 10RB), and a phospho-STAT3 (pSTAT3) responsive reporter gene, as demonstrated in Example 5. Alternatively, HepG2 cells endogenously expressing the IL-22 receptor may be used. Activation of the receptors leads to activation of the STAT3 signaling pathway, which can be measured using a luciferase reporter gene with a STAT3-induced promoter or by assaying pSTAT3, for example. *In vivo* potency may be determined in animal models or in clinical trials, as is known in the art. By way of illustration, Example 10 describes a clinical trial in which IL-22 target engagement biomarkers have been used to demonstrate surprisingly high potency for Derivative 1.

The half maximal effective concentration ($EC_{50}$) value is often used as a measure of the potency of a drug. As this represents the concentration of drug required to produce 50% of the maximal effect, the lower the $EC_{50}$ value, the better the potency. The derivatives for use in the invention suitably have a potency ($EC_{50}$ value) measured using IL-22 receptor-mediated STAT3 activation in cells of below 1.5 nM, below 1.25 nM, below 1 nM, below 0.75 nM, below 0.5 nM, below 0.25 nM or even below 0.1 nM. Advantageously the potency is below 1 nM. The derivatives for use in the invention suitably have a potency ($EC_{50}$ value) measured by assaying pSTAT3 in cells of below 15 nM, below 12 nM, below 10 nM, below 7 nM or even below 5 nM.

The 99% effective concentration ($EC_{99}$) value can also be used as a measure of the potency of a drug. Again, the lower the $EC_{99}$ value, the better the potency. The derivatives for use in the invention suitably have a potency ($EC_{99}$ value) measured using IL-22 receptor-mediated STAT3 activation in cells of below 2000 ng/mL, below 1800 ng/mL, below 1600 ng/mL, below 1400 ng/mL, below 1200 ng/mL, below 1000 ng/mL or even below 900 ng/mL (as shown in Example 5).

Advantageously, the potency of the derivatives of IL-22 may be higher than that of IL-22-Fc fusions. For example, Genentech has reported a 34-fold reduction in *in vitro* potency for its IL-22-Fc fusion, UTTR1147A, compared to hIL-22 (Stefanich et al., Biochem Pharmacol, 2018, 152:224-235). By contrast, covalent attachment of a fatty acid to hIL-22 has been shown to cause only up to a seven-fold reduction in potency (see Derivatives 1 and 2 in Example 1 of WO 2022/238510 and Derivative 1 in the Examples of WO 2021/089875; both incorporated herein by reference). Whilst both IL-22-Fc fusions and the derivatives for use in the present invention may be comparable in terms of their improved half-life over hIL-22 and biological function in at least some settings, the derivatives for use in the invention may have the additional advantage of minimal loss of potency. By way of illustration, Example 10 has demonstrated surprisingly high potency for Derivative 1 in a clinical trial.

The circulatory elimination half-life ($t_{1/2}$) of the derivatives may be determined *in vivo* by administering the derivatives subcutaneously or intravenously in a suitable animal model, such as a mouse, rat or minipig. Suitable methods are described in Example 1 of WO 2021/089875; incorporated herein by reference. By way of a non-limiting example, the derivatives for use in the first or second aspect have a circulatory half-life after subcutaneous or intravenous administration to mice of at least one hour, at least three hours, at least five hours or even at least eight hours. The derivatives may have a circulatory half-life after subcutaneous or intravenous administration to rats of at least three hours, at least five hours, at least eight hours, at least 10 hours or even at least 13 hours. The derivatives may have a circulatory half-life after subcutaneous or intravenous administration to minipigs of at least 25 hours, at least 40 hours, at least 70 hours or even at least 100 hours.

The inventors have also previously found that the derivatives for use in the invention are absorbed rapidly *in vivo*. Advantageously, absorption of the derivatives following subcutaneous dosing may occur faster than that of IL-22-Fc fusions. Mean absorption time is an accurate parameter for measuring uptake because it is independent of dose and maximum plasma concentration following drug administration. It can be calculated based upon mean residence time, i.e. the time that a drug spends in the body prior to elimination once absorption has been completed. The derivatives for use in the invention suitably have a mean absorption time in pigs of below 100 h, below 90 h, below 80 h, below 70 h or even below 60 h (e.g. determined as described in Example 1 of WO 2021/089875; incorporated herein by reference).

The derivatives for use in the invention also have good biophysical properties, such as high physical stability and/or solubility, which may be measured using standard methods in the art.

### IV. Pharmaceutical Compositions Comprising Derivatives of IL-22

Therefore, a pharmaceutical composition comprising a derivative as described or envisaged herein and a pharmaceutically acceptable vehicle may be prepared.

A pharmaceutical composition may comprise any of the different derivatives of IL-22 described or envisaged herein. Suitably, it comprises one of the derivatives of IL-22 identified herein as Derivative 1-14. Advantageously it comprises one of the derivatives of IL-22 identified herein as Derivative 1, 6, 11 or 12.

A derivative as described or envisaged herein, or a pharmaceutical composition comprising the same, will suitably demonstrate increased circulatory elimination half-life compared to hIL-22. Advantageously it will demonstrate increased circulatory elimination half-life compared to hIL-22 by at least 50%, at least 75%, at least 100% or more.

The pharmaceutical compositions may be prepared by combining a therapeutically effective amount of a derivative as described or envisaged herein with a pharmaceutically acceptable vehicle. The formulation of pharmaceutically active ingredients with various excipients is known in the art.

A "therapeutically effective amount" of a derivative as described or envisaged herein is any amount which, when administered to a subject, is the amount of derivative that is needed to treat the disease, disorder or condition or produce the desired effect.

For example, the therapeutically effective amount of derivative used may be from about 0.001 mg to about 1000 mg, and preferably from about 0.01 mg to about 500 mg. It is preferred that the amount of derivative is an amount from about 0.1 mg to about 100 mg, and most preferably from about 0.5 mg to about 50 mg. As a guide, the doses of derivative used in the mice in Example 3 described herein were 0.0125 - 0.5 mg/kg (administered subcutaneously); 0.05 mg/kg was observed as the first therapeutic dose. Examples 6-8 described herein made use of doses in the range of 0.0125 - 0.6 mg/kg (administered subcutaneously); 0.0125 mg/kg was observed as the first therapeutic dose (in Example 7).

A "pharmaceutically acceptable vehicle" as referred to herein, is any known compound or combination of known compounds that are known to those skilled in the art to be useful in formulating pharmaceutical compositions.

The pharmaceutically acceptable vehicle is suitably a liquid; optionally the pharmaceutical composition is in the form of a solution. Liquid vehicles are used in preparing solutions, suspensions, emulsions, syrups, elixirs and pressurised compositions. The derivative for use in the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid vehicles for parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). For parenteral administration, the vehicle can also be an oily ester such as ethyl oleate and isopropyl myristate. Sterile liquid vehicles are useful in sterile liquid form compositions for parenteral administration.

The process for preparing a pharmaceutical composition may thus comprise the usual steps that are standard in the art.


### V. Methods of Therapy

According to the first aspect of the invention, a derivative of IL-22 comprising a fatty acid covalently attached to an IL-22 protein, for use in a method of treating a metabolic, gut and/or liver disease, disorder or condition, said method comprising administering the derivative of IL-22 subcutaneously, is provided.

According to the second aspect of the invention, a derivative of IL-22 comprising a fatty acid covalently attached to an IL-22 protein, for use in a method of treating a metabolic, gut and/or liver disease, disorder or condition, said method comprising administering the derivative of IL-22 intravenously, is provided.

In either aspect, the derivative of IL-22 may be in the form of a pharmaceutical compositions as herein described. A method of treating a subject having a metabolic, gut and/or liver disease, disorder or condition comprising administering subcutaneously or intravenously such a derivative, or a pharmaceutical composition comprising the same, is also provided. Any of the different derivatives of IL-22 described or envisaged herein are expressly included in these aspects of the invention.

Terms such as "treating" and "therapy", as used herein, expressly include the treatment, amelioration or prevention of a disease, disorder or condition.

The derivative of IL-22 or pharmaceutical composition comprising the same may be administered directly into a subject to be treated. It is administered subcutaneously or intravenously, suitably by injection or infusion. Advantageously it is administered subcutaneously and advantageously by injection. Intravenous administration is advantageously by infusion. The derivatives therefore have a clear advantage over Fc fusions in their flexibility of administration because of their smaller size and higher potency. It will be appreciated that administration, into a subject to be treated, of a derivative or pharmaceutical composition comprising the same will result in the increased circulation time compared to hIL-22, and that this will aide in treating a disease, disorder or condition. As above, "treating" also includes ameliorating and preventing a disease, disorder or condition.

Liquid pharmaceutical compositions, which are sterile solutions or suspensions, can be utilised by, for example, subcutaneous or intravenous injection or infusion. The derivative may be prepared as a sterile solid composition that may be dissolved or suspended at the time of administration using sterile water, saline or other appropriate sterile medium.

A derivative or pharmaceutical composition as described or envisaged herein may be administered to any subject in need thereof. A "subject", as used herein, may be a vertebrate, mammal or domestic animal. Hence, derivatives and compositions as used in the invention may be used to treat any mammal, for example livestock (for example, a horse), pets, or may be used in other veterinary applications. Most preferably, the subject is a human being. The derivatives and compositions need not only be administered to those already showing signs of a disease, disorder or condition. Rather, they can be administered to apparently healthy subjects as a purely preventative measure against the possibility of such a disease, disorder or condition in future.

It will be appreciated that derivatives of IL-22 and compositions as described or envisaged herein may be used in a monotherapy (i.e. the sole use of that derivative or composition), for treating a disease, disorder or condition. Alternatively, such derivatives and compositions may be used as an adjunct to, or in combination with, known therapies for treating a disease, disorder or condition.

It will be appreciated that the amount of the derivative of IL-22 that is required is determined by its biological activity, half-life and bioavailability, which in turn depends on the mode of administration, the physiochemical properties of the derivative and composition, and whether it is being used as a monotherapy or in a combined therapy. The frequency of administration will also be influenced by the half-life of the derivative within the subject being treated. Optimal dosages to be administered may be determined by those skilled in the art, and will vary with the particular derivative in use, the strength of the pharmaceutical composition, the mode of administration, and the advancement of the disease, disorder or condition. Additional factors depending on the particular subject being treated will result in a need to adjust dosages, including subject age, weight, gender, diet and time of administration.

Generally, a daily dose of between 0.1 $\mu$g/kg of body weight and 60 $\mu$g/kg of body weight of derivative of IL-22 as described or envisaged herein may be used for treating a disease, disorder or condition, depending upon which derivative or composition is used. More preferably, the daily dose is between 0.1 $\mu$g/kg of body weight and 40 $\mu$g/kg of body weight, more preferably between 0.1 $\mu$g/kg and 30 $\mu$g/kg body weight, and most preferably between approximately 0.1 $\mu$g/kg and 15 $\mu$g/kg body weight.

In a particularly preferred embodiment of the first aspect, where the derivative is for subcutaneous administration, the weekly dose is between approximately 1 $\mu$g/kg and 500 $\mu$g/kg of body weight, more preferably between approximately 1 $\mu$g/kg and 400 $\mu$g/kg body weight and most preferably between approximately 1 $\mu$g/kg and 15 $\mu$g/kg body weight. Further suitable ranges for the weekly dose are between approximately: 1 $\mu$g/kg and 300 $\mu$g/kg, 1 $\mu$g/kg and 200 $\mu$g/kg, 1 $\mu$g/kg and 150 $\mu$g/kg, 1 $\mu$g/kg and 80 $\mu$g/kg, 1 $\mu$g/kg and 50 $\mu$g/kg, 1 $\mu$g/kg and 30 $\mu$g/kg, 1 $\mu$g/kg and 10 $\mu$g/kg, 1 $\mu$g/kg and 7.5 $\mu$g/kg, 1 $\mu$g/kg and 5 $\mu$g/kg, 1 $\mu$g/kg and 2.5 $\mu$g/kg, 1 $\mu$g/kg and 1.25 $\mu$g/kg, 1.25 $\mu$g/kg and 7.5 $\mu$g/kg, 1.25 $\mu$g/kg and 5 $\mu$g/kg, 1.25 $\mu$g/kg and 2.5 $\mu$g/kg, 2.5 $\mu$g/kg and 15 $\mu$g/kg, 2.5 $\mu$g/kg and 10 $\mu$g/kg, 2.5 $\mu$g/kg and 7.5 $\mu$g/kg, 2.5 $\mu$g/kg and 5 $\mu$g/kg, 3 $\mu$g/kg and 100 $\mu$g/kg, 3 $\mu$g/kg and 80 $\mu$g/kg, 3 $\mu$g/kg and 50 $\mu$g/kg, 3 $\mu$g/kg and 30 $\mu$g/kg, 3 $\mu$g/kg and 15 $\mu$g/kg, 3 $\mu$g/kg and 10 $\mu$g/kg, 3 $\mu$g/kg and 5 $\mu$g/kg, 5 $\mu$g/kg and 80 $\mu$g/kg, 5 $\mu$g/kg and 50 $\mu$g/kg, 5 $\mu$g/kg and 30 $\mu$g/kg, 5 $\mu$g/kg and 15 $\mu$g/kg, 5 $\mu$g/kg and 10 $\mu$g/kg, 5 $\mu$g/kg and 7.5 $\mu$g/kg, 10 $\mu$g/kg and 150 $\mu$g/kg, 10 $\mu$g/kg and 100 $\mu$g/kg, 10 $\mu$g/kg and 80 $\mu$g/kg, 10 $\mu$g/kg and 50 $\mu$g/kg, 10 $\mu$g/kg and 15 $\mu$g/kg, 20 $\mu$g/kg and 150 $\mu$g/kg, 20 $\mu$g/kg and 100 $\mu$g/kg, 20 $\mu$g/kg and 80 $\mu$g/kg, 25 $\mu$g/kg and 300 $\mu$g/kg, 25 $\mu$g/kg and 200 $\mu$g/kg, 25 $\mu$g/kg and 150 $\mu$g/kg, 30 $\mu$g/kg and 150 $\mu$g/kg, 30 $\mu$g/kg and 100 $\mu$g/kg, 50 $\mu$g/kg and 350 $\mu$g/kg, 50 $\mu$g/kg and 250 $\mu$g/kg, 50 $\mu$g/kg and 150 $\mu$g/kg, 100 $\mu$g/kg and 400 $\mu$g/kg, 100 $\mu$g/kg and 250 $\mu$g/kg, 200 $\mu$g/kg and 400 $\mu$g/kg, and 200 $\mu$g/kg and 300 $\mu$g/kg, of body weight. Any combination of the end points of these ranges is also envisaged and forms part of the invention.

In a particularly preferred embodiment of the second aspect, where the derivative is for intravenous administration, the weekly dose is between approximately 1 $\mu$g/kg and 400 $\mu$g/kg of body weight, more preferably between approximately 1 $\mu$g/kg and 300 $\mu$g/kg body weight and most preferably between approximately 1 $\mu$g/kg and 15 $\mu$g/kg body weight. Further suitable ranges for the weekly dose are between approximately: 1 $\mu$g/kg and 200 $\mu$g/kg, 1 $\mu$g/kg and 100 $\mu$g/kg, 1 $\mu$g/kg and 80 $\mu$g/kg, 1 $\mu$g/kg and 50 $\mu$g/kg, 1 $\mu$g/kg and 30 $\mu$g/kg, 1 $\mu$g/kg and 10 $\mu$g/kg, 1 $\mu$g/kg and 7.5 $\mu$g/kg, 1 $\mu$g/kg and 5 $\mu$g/kg, 1 $\mu$g/kg and 2.5 $\mu$g/kg, 1 $\mu$g/kg and 1.25 $\mu$g/kg, 1.25 $\mu$g/kg and 7.5 $\mu$g/kg, 1.25 $\mu$g/kg and 5 $\mu$g/kg, 1.25 $\mu$g/kg and 2.5 $\mu$g/kg, 2.5 $\mu$g/kg and 15 $\mu$g/kg, 2.5 $\mu$g/kg and 10 $\mu$g/kg, 2.5 $\mu$g/kg and 7.5 $\mu$g/kg, 2.5 $\mu$g/kg and 5 $\mu$g/kg, 3 $\mu$g/kg and 100 $\mu$g/kg, 3 $\mu$g/kg and 80 $\mu$g/kg, 3 $\mu$g/kg and 50 $\mu$g/kg, 3 $\mu$g/kg and 30 $\mu$g/kg, 3 $\mu$g/kg and 15 $\mu$g/kg, 3 $\mu$g/kg and 10 $\mu$g/kg, 3 $\mu$g/kg and 5 $\mu$g/kg, 5 $\mu$g/kg and 80 $\mu$g/kg, 5 $\mu$g/kg and 50 $\mu$g/kg, 5 $\mu$g/kg and 30 $\mu$g/kg, 5 $\mu$g/kg and 15 $\mu$g/kg, 5 $\mu$g/kg and 10 $\mu$g/kg, 5 $\mu$g/kg and 7.5 $\mu$g/kg, 10 $\mu$g/kg and 150 $\mu$g/kg, 10 $\mu$g/kg and 100 $\mu$g/kg, 10 $\mu$g/kg and 80 $\mu$g/kg, 10 $\mu$g/kg and 50 $\mu$g/kg, 10 $\mu$g/kg and 15 $\mu$g/kg, 20 $\mu$g/kg and 150 $\mu$g/kg, 20 $\mu$g/kg and 100 $\mu$g/kg, 20 $\mu$g/kg and 80 $\mu$g/kg, 25 $\mu$g/kg and 300 $\mu$g/kg, 25 $\mu$g/kg and 200 $\mu$g/kg, 25 $\mu$g/kg and 100 $\mu$g/kg, 30 $\mu$g/kg and 150 $\mu$g/kg, 30 $\mu$g/kg and 100 $\mu$g/kg, 50 $\mu$g/kg and 350 $\mu$g/kg, 50 $\mu$g/kg and 250 $\mu$g/kg, 50 $\mu$g/kg and 150

μg/kg, 100 μg/kg and 300 μg/kg, 100 μg/kg and 250 μg/kg, 150 μg/kg and 300 μg/kg, and 150 μg/kg and 250 μg/kg, of body weight. Any combination of the end points of these ranges is also envisaged and forms part of the invention.

The derivative of IL-22 or composition may be administered before, during or after onset of the disease, disorder or condition. Weekly doses may be given as a single administration (for example, a single weekly injection). Suitable dose ranges for single weekly doses will be the same as those set out above; for example, a single dose may be between approximately: 1 μg/kg and 400 μg/kg, 1 μg/kg and 300 μg/kg, 1 μg/kg and 200 μg/kg, 1 μg/kg and 80 μg/kg, 1 μg/kg and 50 μg/kg, 1 μg/kg and 30 μg/kg, 1 μg/kg and 15 μg/kg, 1 μg/kg and 10 μg/kg, 1 μg/kg and 7.5 μg/kg, 1 μg/kg and 5 μg/kg, 1 μg/kg and 2.5 μg/kg, 1 μg/kg and 1.25 μg/kg, 1.25 μg/kg and 7.5 μg/kg, 1.25 μg/kg and 5 μg/kg, 1.25 μg/kg and 2.5 μg/kg, 2.5 μg/kg and 15 μg/kg, 2.5 μg/kg and 10 μg/kg, 2.5 μg/kg and 7.5 μg/kg, 2.5 μg/kg and 5 μg/kg, 3 μg/kg and 100 μg/kg, 3 μg/kg and 80 μg/kg, 3 μg/kg and 50 μg/kg, 3 μg/kg and 30 μg/kg, 3 μg/kg and 15 μg/kg, 3 μg/kg and 10 μg/kg, 3 μg/kg and 5 μg/kg, 5 μg/kg and 80 μg/kg, 5 μg/kg and 50 μg/kg, 5 μg/kg and 30 μg/kg, 5 μg/kg and 15 μg/kg, 5 μg/kg and 10 μg/kg, 5 μg/kg and 7.5 μg/kg, 10 μg/kg and 150 μg/kg, 10 μg/kg and 100 μg/kg, 10 μg/kg and 80 μg/kg, 10 μg/kg and 50 μg/kg, 10 μg/kg and 15 μg/kg, 20 μg/kg and 150 μg/kg, 20 μg/kg and 100 μg/kg, 20 μg/kg and 80 μg/kg, 25 μg/kg and 300 μg/kg, 25 μg/kg and 200 μg/kg, 30 μg/kg and 150 μg/kg, 30 μg/kg and 100 μg/kg, 50 μg/kg and 350 μg/kg, 50 μg/kg and 250 μg/kg, 50 μg/kg and 150 μg/kg and 100 μg/kg and 250 μg/kg. Advantageously a single weekly dose of between approximately 1 μg/kg and 15 μg/kg body weight (preferably between approximately 1 μg/kg and 10 μg/kg, 1.25 μg/kg and 7.5 μg/kg or 1 μg/kg and 5 μg/kg body weight, such as approximately 1.25 μg/kg, 2.5 μg/kg, 5 μg/kg or 7.5 μg/kg body weight) is given subcutaneously or intravenously (preferably subcutaneously). Alternatively, the derivative or composition may require administration twice or more times during a week, with the above single doses being adjusted accordingly. Doses may alternatively be given daily, every fortnight or once a month. Repeated dosing may be in the 1-7.5 μg/kg body weight range, such as approximately 1.25, 2.5, 5.0 or 7.5 μg/kg body weight. Known procedures, such as those conventionally employed by the pharmaceutical industry (for example, *in vivo* experimentation, clinical trials, etc.), may be used to form specific formulations of the derivatives and compositions according to the invention and precise therapeutic regimes (such as weekly doses of the agents and the frequency of administration).

It may be advantageous to use a loading dose, wherein the first dose administered to the patient is higher than the doses administered subsequently, with the intention to achieve a steady state after the first dose. For example, on a weekly dosage regimen, the first dose in week 1 may be a loading dose that is higher than the doses administered in week 2, week 3 and potentially onwards (as exemplified in Example 10). The loading dose is suitably the weekly dose plus an additional amount of between approximately 0.2 and 4 times the weekly dose, preferably between approximately 0.4 and 2 times the weekly dose, more preferably between approximately 0.6 and 2 times the weekly dose, even more preferably between approximately 0.8 and 2 times the weekly dose, and most preferably between approximately 1 and 2 times the weekly dose. Suitably the loading dose is between approximately 1 μg/kg and 15 μg/kg body weight (preferably between approximately 1 μg/kg and 10 μg/kg, 1 μg/kg and 5 μg/kg, 1.25 μg/kg and 12.5 μg/kg, 1.25 μg/kg and 7.5 μg/kg, 2.5 μg/kg and 15 μg/kg, 2.5 μg/kg and 10 μg/kg or 2.5 μg/kg and 5 μg/kg body weight, such as approximately 2.5 μg/kg, 5 μg/kg, 10 μg/kg or 15 μg/kg body weight). Advantageously the loading dose is double the weekly dose; for example, the loading dose may be 15 μg/kg body weight and the weekly dose may be 7.5 μg/kg body weight. Other suitable examples include a loading dose of 2.5 μg/kg body weight followed by a weekly dose of 1.25 μg/kg body weight, a loading dose of 5 μg/kg body weight followed by a weekly dose of 2.5 μg/kg body weight and a loading dose of 10 μg/kg body weight followed by a weekly dose of 5 μg/kg body weight. In a particularly preferred embodiment, the loading dose is between approximately 2.5 and 15 μg/kg body weight and a recurring weekly dose is between approximately 2.5 and 7.5 μg/kg body weight. Suitably, the loading dose is given in the first week and the weekly doses are given for the rest of the treatment period. The loading dose is suitably given as a single administration (for example, a single subcutaneous injection or single intravenous infusion).

Example 10 provides evidence of a surprisingly high potency for Derivative 1 in man. As described in Example 10, the ascending dose study was originally designed to investigate doses of 3-400 μg/kg body weight. It was never anticipated that doses of less than 3 μg/kg body weight could be therapeutically effective. The low doses of the derivatives described herein that can be administered for therapeutic purposes are therefore both novel and surprisingly advantageous. By comparison, Genentech has disclosed a much higher dose, of 30-90 μg/kg body weight, for its II,-22-Fc fusion, UTTR1147A (Wagner F., et al. Gut 2023; 0:1-11).

Many studies have demonstrated key effects of IL-22 in multiple epithelial injury models in especially lung, liver, intestine, kidney, skin, pancreas and thymus. Mechanistically, several pathways within e.g. anti-apoptosis, proliferation, innate immunity, anti-oxidative stress, anti-fibrosis, and stem cell/progenitor cell recruitment have been well documented to mediate IL-22 effects in studies by multiple investigators. Key mechanistic findings have been further confirmed *in vitro* using human cell lines or in human *ex vivo* models (e.g. primary human intestinal organoids). The strong role of IL-22 in preventing cell death, securing regeneration, and controlling inflammation in epithelial injury is therefore well established.

Many studies are performed by analysing genetic models (IL-22 knock-out or transgenic overexpression) subjected to injury. In these studies, the lack of IL-22 or overexpression of IL-22 will be there at the time of injury. In other studies, IL-22 is neutralised with antibodies at the time of injury and, in some cases, IL-22 is neutralised beyond the acute injury phase (e.g. sub-acutely or well into a regeneration phase). Other studies move closer to a treatment scenario by looking at effects of exogenously administered IL-22. Important to note, when looking holistically at the available literature, is that the different models, whether knock-out, overexpression, IL-22 neutralisation before or after injury or exogenous protein dosing, paint the same picture of IL-22 protecting the injured organs and driving regeneration. This indicates a broad application and a broad time window for IL-22 treatment potential, and also shows why a longer-acting IL-22 protein than hIL-22 is required.

According to the first and second aspect of the invention, however, the derivative of IL-22 (or a pharmaceutical composition comprising the same) is for use in a method of treating a metabolic, gut and/or liver disease, disorder or condition. Any of the different derivatives of IL-22 described or envisaged herein are expressly included in these aspects of the invention. Any of the described dose ranges can be used for any metabolic, gut and/or liver disease, disorder or condition.

The term "metabolic disease, disorder or condition" can mean any disease, disorder or condition that disrupts the body's ability to convert food to energy (i.e. one's metabolism). The metabolic disease, disorder or condition may be obesity, diabetes type 1, diabetes type 2, hyperlipidemia, hyperglycemia or hyperinsulinemia.

The gut disease, disorder or condition may be inflammatory bowel disease (IBD), ulcerative colitis, Crohn's disease, graft-versus-host disease (GvHD), a chemical injury, a viral infection, a bacterial infection or short bowel disease.

The liver disease, disorder or condition may be non-alcoholic fatty liver disease (NAFLD), NASH, cirrhosis, alcoholic hepatitis, acute liver failure, chronic liver failure, acute-on-chronic liver failure (ACLF), acetaminophen induced liver toxicity, acute liver injury, sclerosing cholangitis, biliary cirrhosis or a pathological condition caused by surgery or transplantation.

A method of treating a subject having a metabolic, gut and/or liver disease, disorder or condition, such as one or more as mentioned above, with a derivative of IL-22 as described or envisaged herein, or a pharmaceutical composition comprising the same, is also provided.

There is no restriction on which derivative of IL-22 or composition as described herein should be administered to which patient. Rather, it is intended that any of the derivatives and compositions described herein can be administered to any patient as described herein.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made to the Examples, which are not intended to limit the invention in any way.

## EXAMPLES

Derivative 1 (see Table 3 and Figure 2) is a novel long-acting IL-22 analogue under development for chronic treatment of gut and liver diseases, disorders or conditions. The intended clinical route of administration is either subcutaneous or intravenous. A dosage regimen in man has been devised based upon the following results of animal studies.

### Example 1 - Subcutaneous Toxicokinetic (TK) Study in Minipigs

*Objective*

This study aimed to establish the TK profile of Derivative 1 when administered once weekly by subcutaneous injection to Göttingen Minipigs for six weeks followed by a four-week recovery period for selected animals.

*Overview*

The minipig was selected as the test model since it has demonstrated pharmacological responsiveness to treatment in PK/PD minipig studies and single and four subcutaneous doses of Derivative 1 were well-tolerated in the same studies and because of its well accepted suitability in this type of study. Subcutaneous injection was chosen in order to comply with one of the intended human routes of administration. The doses were selected on the basis of the results of a Dose Range Finding study in minipigs where 9.48 mg/kg was found not to be tolerated over four weeks, whereas 1 mg/kg did not result in any adverse finding. Furthermore, one animal tolerated the once weekly dosing of 9.48 mg/kg for two weeks and, due to findings in the other animals, the dose was reduced to 3 mg/kg for the remaining part of the study, which was tolerated.

*Methods*

*(i) Animals*

40 Göttingen specified pathogen-free (SPF) minipigs (20 males and 20 females) were obtained from Ellegaard Göttingen Minipigs A/S, Denmark. The animals were 3-4 months old and had a body weight of 7-9 kg on arrival. The animals were housed according to the relevant guidelines (individually for males and group-housing for females) and fed twice daily with a minipig diet (SMP (E) SQC from Special Diets Services, UK) in an amount of approximately 125 g per animal per meal. From Day 1 the dosed animals (Groups 2-4; see Table 4) received an additional 50 g of diet in the morning. All animals had *ad libitum* access to domestic quality drinking water.

On the day of arrival, the minipigs were allocated randomly to four groups (Groups 1-4), using a randomisation scheme. In connection with the allocation, any littermates were distributed evenly between the groups.

A pre-treatment period of approximately three weeks (including an acclimatisation period of five days) was allowed during which the animals were observed daily in order to reject animals in poor condition.

Approximately one week prior to study start, the animals were assessed for weight and, if the group means were very uneven, the animals were re-allocated to achieve homogeneity of mean group weight. Data available from pre-treatment observations, clinical signs and laboratory investigations were also taken into account when re-allocating animals.

Prior to the start of treatment, the two injection sites, one on each side, were clipped and marked on the neck region of the animals. The injection sites were marked in each corner. The tattooed areas were approximately 2x5 cm, as illustrated below.

Cranial

Animal Left | Animal Right

Site 1    •   |   •    Site 2

Caudal

Prior to tattooing, anaesthesia was achieved by an intramuscular injection in the left hind leg (1.0 mL/10 kg body weight) of a mixture of Zoletil 50®Vet. (125 mg tiletamine and 125 mg zolazepam; Virbac, France), 20 mg xylazine/mL (6.25 mL), 100 mg ketamine/mL (1.25 mL) and 10 mg butorphanol/mL (2.5 mL). If a supplement was necessary, usually 1/3 of the original dose was given. However, individual needs were taken into consideration.

*(ii) Dose formulation preparation*

A quality control analysis of Derivative 1 was carried out using standard procedures to establish its identity, purity and stability. Once characterised, Derivative 1 was supplied as a 9.25 mg/mL stock solution in phosphate buffered saline ((PBS): 1.96 mM $KH_2PO_4$, 8.05 mM $Na_2HPO_4$, 140 mM NaCl, pH 7.4). The dose formulations were prepared by diluting the stock solution in sterile filtered PBS to the required concentrations. Analysis of a test sample was performed by high pressure liquid chromatography (HPLC) with ultraviolet (UV)-detection. To be considered acceptable, the concentration of each formulation needed to be within $\pm 10\%$ of nominal.

*(iii) Treatment*

The groups, dose levels and animal numbers (from 1-40) for the main study and recovery period were as follows:

*Table 4: Treatment schedule (\* Day 1; \*\*Days 15, 22, 29 and 36)*

| Group | Dose (mg/kg) | Dose concentration (mg/mL) | Animal # (Main study) | | Animal # (Recovery) | |
|---|---|---|---|---|---|---|
| | | | Male | Female | Male | Female |
| 1 | 0 (vehicle) | 0 | 1-3 | 4-6 | 25-26 | 27-28 |
| 2 | 0.3 | 0.75 | 7-9 | 10-12 | 29-30 | 31-32 |
| 3 | 1.0 | 2.5 | 13-15 | 16-18 | 33-34 | 35-36 |
| 4 | 3.0* | 7.5 | 19-21 | 22-24 | 37-38 | 39-40 |
| 4 | 0.6** | 1.5 | 19-21 | 22-24 | 37-38 | 39-40 |

The first day of treatment was designated Day 1.

The once weekly dose for Groups 1-3 was given by subcutaneous injection according to the most recent body weight data to both the main and recovery animals on Days 1, 8, 15, 22, 29 and 36.

Group 4 main and recovery animals were given the once weekly dose by subcutaneous injection according to the most recent body weight data on Days 1, 15, 22, 29 and 36. They were allowed a wash out period in week 2 due to clinical signs.

Treatment was performed in Site 1 (left) on Days 1, 15 and 29 and Site 2 (right) on Day 8, 22 and 36.

The dose volume was 0.40 mL/kg. Due to the volume and the size of the animals, dosing took place using a butterfly needle to allow for a slow injection rate as necessary.

The weight of dose formulation for each group before and after dosing was documented. After dosing, the amount of dose formulation used for each group was compared with the predicted daily usage.

For the recovery animals, following the six-week treatment period, a treatment-free period of four weeks was introduced.

*(iv) Blood sampling & observations*

Blood samples for TK analysis were drawn from the jugular vein/bijugular trunk at certain timepoints during the study. Specifically, on the first day of dosing (Day 1), blood sampling for TK was performed at the following time points on all animals: 1.5, 4, 8, 12, 24, 48, 72 and 168 hours (the latter just before dosing Day 8). Furthermore, on Day 36: pre-treatment and 1.5, 4, 8, 12, 24, 48, 72 and 168 hours after dosing.

Animals were monitored daily throughout the study for clinical observations including injection site reactions and behavioural changes. The animals were weighed weekly at the same time of day and body weight gain calculated.

*(v) Bioanalysis*

Minipig serum samples were analysed to quantitatively determine Derivative 1 using a fully validated assay (the commercially available "V-PLEX Human IL-22 Kit" from Meso Scale Discovery (MSD)). In brief, a 96-Well Small Spot plate, pre-coated with a capture antibody against human IL-22 on well-defined single spots, was provided in the kit (MSD Multi-Array®). After washing (3x with 150 $\mu$l/well wash buffer), 50 $\mu$l/well of calibration standards, quality control samples and unknown samples was added to the respective wells. After incubation (2h, 600 rpm, room temperature), the plate was washed (as before) and 25 $\mu$l/well of a SULFO-TAG® conjugated detection antibody was added. After incubation (as before), the plate was washed again (as before) and 150 $\mu$l/well of 2x Read Buffer T (MSD) was added to the respective wells. The plate was analysed using the QuickPlex SQ 120 reader (MSD), which generates electrochemiluminescent (ECL) signals. The signals of the calibration standards, quality control samples and unknown samples were converted into concentrations using the regression curve calculated based on the signals from the calibration samples. Calculations were performed using the Workbench® software from MSD and/or Watson LIMS.

A TK evaluation of the results of the analysis was carried out using the commercial and validated software, Phoenix WinNonlin (version 8.3 or later). All critical operations and methods were performed and documented according to current written local standard operating procedure unless otherwise noted. The TK analysis consisted of the assessment of standard parameters including maximum concentration ($C_{max}$), time to obtain $C_{max}$ ($T_{max}$), time of last measurable concentration ($T_{last}$), t½, area under the concentration time-curve to the last measurable concentration ($AUC_{last}$), AUC of dosing interval ($AUC_{tau}$), AUC extrapolated as a percentage of total ($AUC_{\%extrap}$), AUC extrapolated to 168 h ($AUC_{0-168h}$), AUC extrapolated to infinity ($AUC_{inf}$), and the drug accumulation ratio ($R_{ac}$) if data permitted. Dose corrected values (linearity) of exposure (AUC and $C_{max}$) were also given, when possible. Gender specific estimates and a pooled estimate were generated for each of the TK parameters. Other TK parameters were assessed as considered appropriate.

Data were processed to give group mean values and standard deviations where appropriate. The statistical analysis was performed using Instem Provantis® (version 9.3.0.0).

*Results*

Table 5 shows the major TK parameters after once-weekly subcutaneous administration of Derivative 1 to minipigs.

*Table 5: TKparameters in minipigs*

| Parameter | Unit | Group 2 (0.3 mg/kg) | | Group 3 (1.0 mg/kg) | | Group 4 (3.0 mg/kg) | |
|---|---|---|---|---|---|---|---|
| | | Male | Female | Male | Female | Male | Female |
| $AUC_{\%extrap}$ | % | 34.9 | 25.2 | 46 | 41.4 | 25.5 | 29.3 |
| $AUC_{0.168h}$ | h*ng/mL | 62800 | 67600 | 194000 | 244000 | 654000 | 737000 |
| $AUC_{last}$ | h*ng/mL | 62800 | 67600 | 194000 | 244000 | 654000 | 737000 |
| $C_{max}$ | ng/mL | 703 | 893 | 2090 | 3110 | 8030 | 8990 |
| t½ | h | 118 | 85.9 | 171 | 91.9 | 86 | 94.5 |
| $T_{last}$ | h | 168 | 168 | 168 | 168 | 168 | 168 |
| $T_{max}$ | h | 10.4 | 7.2 | 5.6 | 6.4 | 9.6 | 7.2 |

The results show that average serum concentrations of Derivative 1 following subcutaneous administration reached maximum concentrations ($T_{max}$) at 5.6-10.4 hours post dose. Serum concentrations were detectable above the below limit of quantification (BLOQ; 0.0488 ng/mL) until 168 hours post dose ($T_{last}$) at all dose levels. Following absorption, Derivative 1 was eliminated with a mean elimination half-life ($t_{1/2}$) of 85.9-171 hours. The increase in $C_{max}$ was proportional to dose in both males and females after a single subcutaneous dose, when comparing lowest and highest dose levels.

*Conclusions*

These data from a six-week repeat dose study with subcutaneous administration show exposure to Derivative 1 in minipigs. The observed long elimination half-life ($t_{1/2}$) is supportive of a compound suitable for once-weekly dosing in humans.

**Example 2** - **Bioavailability Study in Minipigs**

*Objective*

This study aimed to establish the PK parameters and bioavailability of Derivative 1 from a single-dose subcutaneous to intravenous cross-over study in Göttingen Minipigs.

*Overview*

The minipig was selected as the test model for the same reasons as provided in Example 1. Subcutaneous injection and intravenous infusion were chosen in order to comply with both of the intended human routes of administration.

*Methods*

The methods used were identical to those described in Example 1. The only difference was the additional PK parameters measured for the intravenous route of administration, which were the apparent volume of distribution during the terminal phase ($V_z$), the drug clearance rate (CL) and bioavailability (i.e. the percentage of the administered dose that reached the systemic circulation; F).

*Results*

Tables 6A and 6B show the PK and bioavailability parameters after intravenous or subcutaneous administration of Derivative 1 to minipigs.

*Table 6A: PK parameters in minipigs (IV = intravenous; SC = subcutaneous)*

| Dose (mg/kg) | Sex | Route | Animal No | $C_{max}$ (ng/mL) | $T_{max}$ (h) | $T_{last}$ (h) | $AUC_{last}$ (h*ng/mL) | $AUC_{INF}$ (h*ng/mL) |
|---|---|---|---|---|---|---|---|---|
| 0.1 | M | IV | 3 | 203 | 1 | 192 | 3410 | 3520 |

(continued)

| Dose (mg/kg) | Sex | Route | Animal No | $C_{max}$ (ng/mL) | $T_{max}$ (h) | $T_{last}$ (h) | $AUC_{last}$ (h*ng/mL) | $AUC_{INF}$ (h*ng/mL) |
|---|---|---|---|---|---|---|---|---|
| | | | 5 | 171 | 1 | 192 | 3530 | 3680 |
| | | | Mean | 187 | 1 | 192 | 3470 | 3600 |
| 0.1 | F | IV | 4 | 199 | 1 | 192 | 3870 | 3940 |
| | | | 8 | 209 | 1 | 192 | 2630 | 2660 |
| | | | Mean | 204 | 1 | 192 | 3250 | 3300 |
| 0.5 | M | SC | 3 | 110 | 8 | 168 | 9810 | 12000 |
| | | | 5 | 98.9 | 4 | 168 | 9560 | 10300 |
| | | | Mean | 104 | 6 | 168 | 9680 | 11100 |
| 0.5 | F | SC | 4 | 130 | 4 | 168 | 8000 | 8710 |
| | | | 8 | 198 | 4 | 168 | 7840 | 7860 |
| | | | Mean | 164 | 4 | 168 | 7920 | 8280 |

*Table 6B: PK parameters in minipigs cont. (IV = intravenous; SC = subcutaneous)*

| Dose (mg/kg) | Sex | Route | Animal No | $AUC_{0-168h}$ (h*ng/mL) | T½ (h) | Vz (mL/kg) | CL (mL/h/kg) | F (%) |
|---|---|---|---|---|---|---|---|---|
| 0.1 | M | IV | 3 | 3370 | 44.9 | 1840 | 28.4 | |
| | | | 5 | 3470 | 47.8 | 1880 | 27.2 | |
| | | | Mean | 3420 | 46.4 | 1860 | 27.8 | |
| 0.1 | F | IV | 4 | 3830 | 34.6 | 1270 | 25.4 | |
| | | | 8 | 2610 | 36.1 | 1960 | 37.7 | |
| | | | Mean | 3220 | 35.4 | 1610 | 31.5 | |
| 0.5 | M | SC | 3 | 9810 | 68.6 | | | 67.9 |
| | | | 5 | 9560 | 41.2 | | | 55.9 |
| | | | Mean | 9680 | 54.9 | | | 61.9 |
| 0.5 | F | SC | 4 | 8000 | 44.2 | | | 44.2 |
| | | | 8 | 7840 | 17.7 | | | 59.2 |
| | | | Mean | 7920 | 30.9 | | | 51.7 |

The results show that Derivative 1, after a single intravenous dose of 0.1 mg/kg, was eliminated with a two-compartmental decay, and the (mean) terminal elimination half-life ($t_{1/2}$) was 46.4 h in males and 35.4 h in females.

After a single subcutaneous dose to minipigs, maximum concentrations of Derivative 1 were reached ($T_{max}$) at four hours post-dose in all animals except Animal No. 3, in which the maximum concentration was observed at eight hours post-dose. Serum concentrations after a single subcutaneous dose of 0.5 mg/kg were detectable above the lower limit of quantification (LLOQ) until 168 hours post-dose ($T_{last}$). Following absorption, Derivative 1 was eliminated with a terminal elimination half-life ($t_{1/2}$) ranging from 18-69 hours after subcutaneous administration.

After intravenous administration, the (mean) maximum concentration ($C_{max}$) ranged from 187-204 ng/mL at 0.1 mg/kg and, after subcutaneous administration, 104-164 ng/mL at 0.5 mg/kg. The corresponding $AUC_{INF}$ was 3300-3600 h*ng/mL at 0.1 mg/kg intravenous and 8280-11100 h*ng/mL at 0.5 mg/kg subcutaneous.

The bioavailability (F) after subcutaneous administration was intermediate, on average 62% in male and 52% in female minipigs.

*Conclusions*

These data show the bioavailability of Derivative 1 in minipigs. The bioavailability after subcutaneous administration was intermediate, on average 57%, with no major sex differences.

## Example 3 - Therapeutic Effect in Colitis Mouse Model

*Objective*

This study aimed to evaluate the effect of Derivative 1 on intestinal parameters in a dextran sodium sulphate (DSS)-induced colitis mouse model.

*Methods*

*(i) Animals*

60 female 10-week old C57Bl/6JRj mice with a body weight of approximately 20-25 g were acquired from Janvier (France) and acclimatised for one week prior to study start in group housing (five per box). Animals were housed according to standard procedures, fed regular chow and tap water and observed daily for any signs of ill health. On the day prior to study start, mice were randomised according to body weight into six groups (n=10 per group) and baseline measurements including body weight were taken.

*(ii) Induction of colitis*

On Day 1 of the study, acute intestinal inflammation (colitis) was induced by dissolving DSS in drinking water (2.5% (w/v)) and administering to all mice except controls. This was repeated once a day for six days, with a return to regular drinking water on Days 7-11. Control mice were administered regular drinking water for the entire study period. Body weight and food and water intake of all mice were measured daily on Days 1-10. The disease activity index was determined by scoring changes in weight loss and rectal bleeding on a daily basis on Days 1-10.

*(iii) Dose formulation preparation*

Derivative 1 was prepared as a 12 mg/mL stock solution in PBS in a clean laboratory at room temperature. On testing, the stock solution was found to be 88.6% pure and hence contained 10.6 mg/mL of Derivative 1. The stock solution was diluted in sterile filtered PBS to provide dosing solutions at concentrations of 0.01, 0.03, 0.08 and 0.25 mg/mL. These were stored at 4 °C until needed. PBS was also used as a control vehicle and was prepared according to standard procedures. 2 mL/kg of Derivative 1 or PBS was administered subcutaneously to the mice every morning on Days 1-10 of the study, as detailed in Table 7.

*Table 7: Groups of mice in study*

| Mouse Group | | | Compound | |
|---|---|---|---|---|
| # | Name | Model | Name | Dose of Derivative 1 (mg/kg) |
| 1 | No DSS vehicle | No DSS control | PBS | n/a |
| 2 | Vehicle | DSS | PBS | n/a |
| 3 | 0.0125 mg/kg | DSS | Derivative 1 | 0.0125 |
| 4 | 0.05 mg/kg | DSS | Derivative 1 | 0.05 |
| 5 | 0.15 mg/kg | DSS | Derivative 1 | 0.15 |
| 6 | 0.5 mg/kg | DSS | Derivative 1 | 0.5 |

*(iv) Bioanalysis*

On Day 11, all mice were terminated by cardiac puncture under isoflurane anaesthesia.
The gut was dissected, flushed with ice cold saline and the content gently removed. The large intestine was then sampled using systematic random uniform sampling (SURS) principles into four slabs and placed in a multi-cassette.

The tissue was infiltrated in formalin (Tissue-Tek VIP®; Sakura) overnight and subsequently embedded in blocks of paraffin. All tissue slabs were placed in such a way that identification of individual slabs was possible at a later stage. The paraffin blocks were trimmed and 5 $\mu$m top sections were cut and mounted on SuperFrost® Plus object glasses (Thermo Fisher Scientific). Another section was cut with a 500 $\mu$m distance to the top section, thus giving rise to a total of eight colon sections from each animal.

Stereological volume estimation was performed using the newCAST system (Visiopharm) on scanned hematoxylin and eosin-stained slides. Total colon volume, volume of mucosa and submucosa, muscularis and inflamed tissue were estimated by point counting using a grid system of appropriate size where all points hitting the structure of interest were counted. The number of points hitting the structure of interest was converted into volume according to the following mathematical relationship:

$$Vol_{ref} = \sum p \cdot A(p) \cdot t$$

where A(p) is the area per point, p is the total number of points hitting the structure of interest and t is the distance between sections.

*Results*

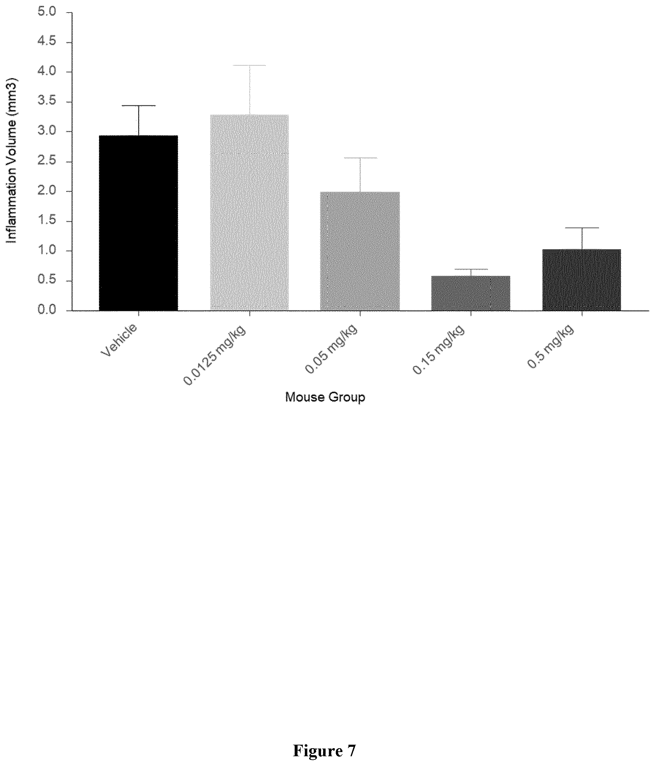

Figure 7 compares colonic inflammation volume for mice in Groups 2-5. The mice in Group 2 (administered only the PBS vehicle) were characterised by a DSS-induced inflamed colon (having a mean volume of around 3 mm$^3$). A reduction in colonic inflammation volume was observed with treatment in Groups 4-6 (dosed with at least 0.05 mg/kg Derivative 1). No reduction, however, was observed in Group 3 (dosed with 0.0125 mg/kg Derivative 1).

*Conclusions*

A reduction in colonic inflammation volume was achieved in a murine model of colitis after subcutaneous administration of Derivative 1. The first therapeutic effect was observed at a dose of 0.05 mg/kg.

**Example 4 - Subcutaneous PK Study in Mice**

*Objective*

This study aimed to establish a PK profile of Derivative 1 when administered in a single dose by subcutaneous injection to C57BL/6J mice.

*Methods*

*(i) Animals*

48 male 10-week old lean C57Bl/6JRj mice were acquired from Janvier (France) and acclimatised for two weeks prior to study start in group housing (three per box). Animals were housed according to standard procedures, fed regular chow and tap water and observed daily for any signs of ill health. 10 days prior to study start, mice were randomised according to body weight into four groups (n=12 per group) and baseline measurements including body weight were taken.

The day before dosing, each animal was shaved at the planned injection site in order to better monitor the injection site reaction.

*(ii) Dose formulation preparation*

Derivative 1 was prepared as a 10 mg/mL stock solution in PBS in a clean laboratory at room temperature. The stock solution was diluted in sterile filtered PBS to provide dosing solutions at concentrations of 0.01, 0.05, 0.15 and 0.5 mg/mL. These were stored at 4 °C until needed. A single 2 mL/kg dose of Derivative 1 was administered subcutaneously in the neck of each mouse on the morning of Day 0 of the study, as detailed in Table 8.

*Table 8: Groups of mice in study*

| Mouse Group | | Compound | |
|---|---|---|---|
| # | Name | Name | Dose of Derivative 1 (mg/kg) |
| 1 | 0.02 mg/kg | Derivative 1 | 0.02 |
| 2 | 0.1 mg/kg | Derivative 1 | 0.1 |
| 3 | 0.3 mg/kg | Derivative 1 | 0.3 |
| 4 | 1 mg/kg | Derivative 1 | 1 |

*(iii) Blood sampling & observations*

At 1, 2, 4 and 8 hours post-dosing, a 75 $\mu$l blood sample was collected from the tail vein of three mice in each group (rotating around all 12 mice in the group across the four timepoints) into a Microvette tube of the appropriate dimension, mixed by inversion five times and allowed to clot at room temperature. Blood was centrifuged at room temperature at 3000 g for 10 min.

The harvested serum samples (at least 25 $\mu$l) were kept in serum separator tubes at -70 °C until required for PK analysis. This serum sampling was repeated on each of Days 1-4.

Body weight was measured in the morning on Days 0, 1, 3 and 4. Animals were observed daily for any clinical signs of ill health including injection site reactions such as bruising, erythema, swelling and crust formation.

*(iv) Bioanalysis*

PK parameters were calculated based on quantitative data using PKSolver. Data were analysed by noncompartmental analysis (NCA).

Mouse serum samples were also analysed to quantitatively determine Derivative 1 using the commercially available "V-PLEX Human IL-22 Kit" from MSD, as described in Example 1.

Single-timepoint continuous data were fitted to a one-factor linear regression model with the treatment groups as categorical, independent (predictor) variables and Dunnett's test was used to compare treatments to control. Continuous data with repeated sample collection over time were fitted to a two-factor linear regression model with the treatment groups and time points as categorical, independent (predictor) variables with interaction. Dunnett's test was again used to compare treatments to control for each timepoint. Data from categorical endpoints, such as histopathological scoring values, were partitioned into a 2x2 contingency table containing responders and non-responders in control and treatment groups. Fisher's exact test assumed that contingency table row and column totals were fixed, sampling was random, and observations could be classified only into one cell. The reported p-values from all pairwise comparisons were adjusted using the Bonferroni correction.

*Results*

Table 9 shows the PK parameters after a single subcutaneous administration of Derivative 1 to mice.

*Table 9: PK parameters in mice*

| Dose (mg/kg) | $C_{max}$ (ng/mL) | $T_{max}$ (h) | $t_{1/2}$ (h) | $AUC_{INF}$ (h*ng/mL) |
|---|---|---|---|---|
| 0.05 | 1.12 | 2 | * | 22.7 |
| 0.1 | 9.77 | 4 | 7.9 | 144.17 |
| 0.3 | 29 | 2 | 7.6 | 475.73 |
| 1 | 127.47 | 2 | 9.1 | 2101.03 |

As can be seen in Table 9, a single subcutaneous dose of 0.05 mg/kg Derivative 1 resulted in a $C_{max}$ of 1.12 ng/mL and an $AUC_{INF}$ of 22.7 h*ng/mL in mice. This was also the dose bringing about the first therapeutic effect observed in a murine acute DSS model in Example 3.

*Conclusions*

These data from a single dose mouse PK study show exposure to Derivative 1 in mice. Serum concentrations of Derivative 1 after a single subcutaneous administration to mice increased in a dose-dependent manner with an elimination half-life ($T_{1/2}$) of on average 8.2 hours.

**Example 5 - *In Vitro* STAT3 Potency Assay**

*Objective*

This study aimed to determine the *in vitro* potency of Derivative 1.

*Overview*

Derivative 1 is able to bind with specific receptors that are overexpressed on the surface of BHK cells. Consequently, a luciferase reporter can be activated and detected using a One-Gio® luciferase assay detection kit (Promega, US), according to manufacturer's instructions.

*Methods*

In this assay, 100 μl/well of BHK cells that overexpress IL10RB and IL22RA (hereinafter "BHK IL22 cells") were seeded at a viable cell density of $2\times10^5$ cells/mL in a 96-well cell culture plate and incubated under cover at 37 °C with 5% $CO_2$ for 20-28 hours.

Following incubation, all medium was removed from each well and 100 μl/well of serially diluted reference standard (i.e. a fixed batch of Derivative 1), assay control and samples added.

The assay control was an independent dilution of the reference standard. The plate was incubated under cover for 3-5 hours at 37 °C with 5% $CO_2$.

Following incubation, 100 μl/well of One-Gio® solution was added and the plate incubated in the dark for 5-20 min. The reaction solution was mixed at least four times by reverse pipetting. 100 μl/well of the reaction solution was then transferred from the culture plate to a 96-well white plate. The plate was placed in a plate reader (Molecular Devices SpectroMax M5e) and the luminescence measured.

By way of explanation, upon binding to IL-22 and IL-10 receptors located on the BHK IL22 cell surface, an intracellular luciferase reporter gene under the control of a STAT3 driven promoter was activated. The potency of Derivative 1 was determined by monitoring the activation of luciferase reporter within the BHK IL22 cells using the One-Gio® luciferase assay system. The intensity of luminescence was proportional to the potency of Derivative 1 within each well.

The assay was repeated so as to ensure accuracy and validity.

Key PD parameters measured were $EC_{99}$, the slope factor of the curve (hill slope), peak (minimum) effect ($E_{min}$) and peak (maximum) effect ($E_{max}$).

The dose response curves (luminescence versus Derivative 1 concentration) were fitted to a 4PL model. The results were reported as Relative Potency calculated using the $EC_{50}$ values of the reference standard versus the Derivative 1 samples.

*Results*

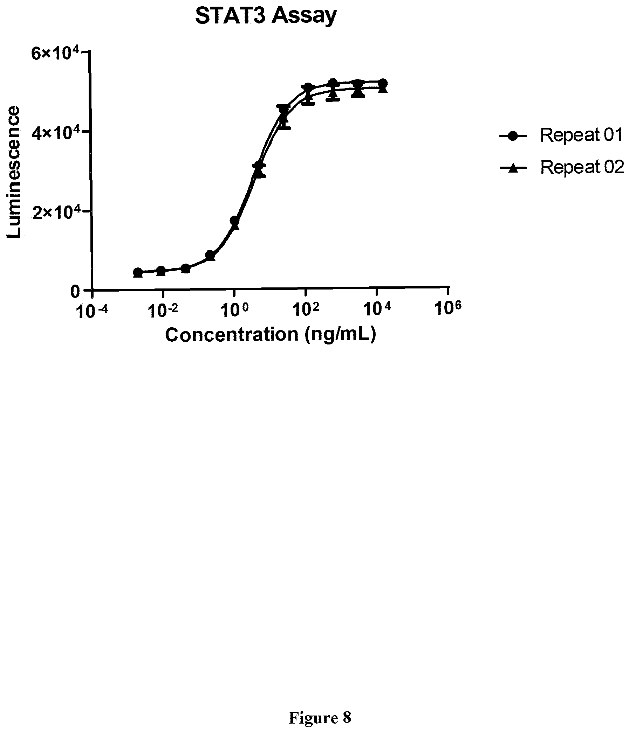

Figure 8 shows *in vitro* STAT3 activation in an IL-22 receptor reporter BHK cell line. As the concentration of Derivative 1 administered to cells increased, so the luminescence of the luciferase reporter increased in a sigmoidal fashion. The two repeats of the assay showed virtually identical results, indicating accuracy and validity.

Table 10 shows the key PD parameters from the *in vitro* STAT3 curves for Derivative 1.

*Table 10: Key PD parameters in a BHK IL22 cell line*

|  | **Repeat 01** | **Repeat 02** |
|---|---|---|
| **$EC_{99}$** | 814 (511-1298) | 952 (364-2487) |
| **Hill slope** | 0.856 (0.783-0.929) | 0.8426 (0.697-0.988) |
| **$E_{min}$** | 4664 (3900-5428) | 4590 (3095-6086) |

(continued)

|  | Repeat 01 | Repeat 02 |
|---|---|---|
| $E_{max}$ | 52002 (51297-52706) | 50367 (48966-51767) |

As per Table 10, the average $EC_{99}$ for two technical replicates of duplicate measures was found to be 883 ng/mL. This shows that Derivative 1 was active in the nanomolar range in this sensitive reporter cell line for IL-22 receptor activation.

*Conclusions*

Derivative 1 was shown to be potent in binding IL10RB and IL22RA on the surface of BHK cells *in vitro*. Derivative 1 therefore showed potency-consistent full activity at the average exposure predicted to be reached in humans following subcutaneous and intravenous administration (as described in Example 9).

**Example 6 - PD Effect in Diabetes Mouse Model**

This study aimed to evaluate the effect of Derivative 1 on blood glucose in db/db mice. These mice are used to model phases 1 to 3 of diabetes type 2 and obesity.

*Methods*

*(i) Animals*

20 7-8-week old C57BKS db/db mice were acquired from Charles River (Germany) and acclimatised for 18 days prior to study start in group housing (10 per cage). Animals were housed according to standard procedures. Two weeks after arrival, each mouse was microchipped and blood glucose was measured. Three days prior to study start, mice were randomised into two groups (n=10 per group), individually caged and blood glucose was again measured. Food intake was measured daily up to study start.

*(ii) Dose formulation preparation*

Derivative 1 was prepared as a 0.125 mg/mL dosing solution in PBS. A PBS vehicle (pH 7.4 with 70 ppm polysorbate 20) was used as a control and was prepared according to standard procedures.

*(iii) Treatment*

4 mL/kg of Derivative 1 or PBS was administered subcutaneously to the mice, as detailed in Table 11, on Days 0-16 of the study. This occurred at 10am each day, except on Day 16, when it occurred at 7am.

*Table 11: Groups of mice in study*

| Group | Formulation | Dose volume (mL/kg) | Dose (mg/kg) | Concentration (mg/mL) |
|---|---|---|---|---|
| 1 | Vehicle | 4.0 | n/a | n/a |
| 2 | Derivative 1 | 4.0 | 0.5 | 0.125 |

Body weight (prior to dosing) and food intake were measured daily. Blood glucose was also measured daily prior to dosing (t = 0 min) and then after dosing (t = 1, 2 and 6 h).

*Results*

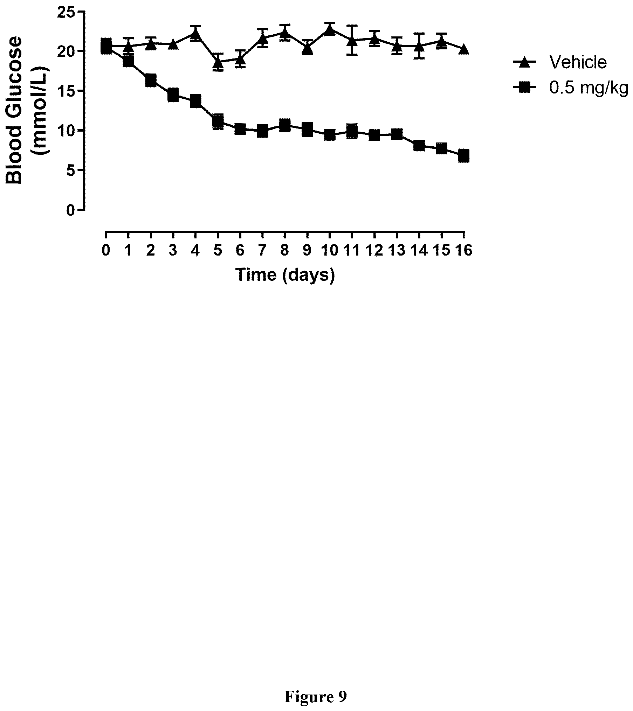

Figure 9 compares blood glucose levels over time for the mice in Groups 1 and 2. The mice in Group 1 (administered only the PBS vehicle) were characterised by high blood glucose, which persisted over the time course. A progressive and significant reduction in blood glucose levels over the time course was observed with treatment in Group 2 (dosed with 0.5 mg/kg Derivative 1).

*Conclusions*

A reduction in blood glucose was achieved in a murine model of diabetes (type 2) and obesity after subcutaneous administration of Derivative 1. This supports the use of Derivative 1 and other derivatives as described herein in the management of these conditions. This is significant, as type 2 diabetes is by far the most common type of diabetes.

Patients with type 2 diabetes either do not make enough insulin or do not make insulin that the body can use properly. The cells in the body become resistant to insulin, making a greater amount of insulin necessary to keep blood glucose levels within a normal range. Eventually, the pancreas can wear out from producing extra insulin, and it may start making less and less. Type 2 diabetes can usually be managed through diet, exercise and self-monitoring blood glucose, at least in the first few years following diagnosis. However, type 2 diabetes is a progressive condition and most people will need to take tablets and/or inject insulin after living with it for five to 10 years.

Given the findings in the study at hand, Derivative 1 and other derivatives as described herein may offer a new therapeutic regime for this condition.

**Example 7 - Therapeutic Effect in Diet-Induced Obesity Mouse Model**

*Objective*

This study aimed to evaluate the effect of four weeks of treatment with Derivative 6 on body weight in male diet-induced obesity mice. A parallel study aimed to evaluate the effect of four weeks of treatment with Derivative 1 on body weight and fasting plasma insulin in male diet-induced obesity mice.

*Methods*

*(i) Animals*

70 male six-week old C57Bl/6JRj mice were acquired from Janvier (France), housed according to standard procedures and fed a 60% high fat diet (SSNIFF, Germany) for 29 weeks until study start, to achieve rapid weight gain. Two weeks prior to study start, the mice were single housed. One week prior to study start, mice were randomised according to body weight into seven groups (n=10 per group) and baseline measurements including body weight were taken. The high fat diet was maintained throughout the study period.

*(ii) Dose formulation preparation*

Derivative 1 was prepared as 0.01, 0.03 and 0.06 mg/mL dosing solutions in PBS. Derivative 6 was prepared as 0.0025, 0.01 and 0.03 mg/mL dosing solutions in PBS. These were stored for up to three days at 4 °C until needed. PBS was also used as a control vehicle and was prepared according to standard procedures.

*(iii) Treatment*

5 mL/kg of Derivative 1, Derivative 6 or PBS was administered subcutaneously to the mice in the afternoon of each of Days 0-28 of the study, as detailed in Table 12. Body weight was measured daily.

*Table 12: Groups of mice in study*

| Group | Formulation | Dose volume (mL/kg) | Dose (mg/kg) |
|---|---|---|---|
| 1 | Vehicle | 5.0 | n/a |
| 2 | Derivative 6 | 5.0 | 0.0125 |
| 3 | Derivative 6 | 5.0 | 0.05 |
| 4 | Derivative 6 | 5.0 | 0.15 (as a titration - 0.05 mg/kg on Day 1, 0.1 mg/kg on Day 2, 0.15 mg/kg on Day 3) |
| 5 | Derivative 1 | 5.0 | 0.05 |
| 6 | Derivative 1 | 5.0 | 0.15 |
| 7 | Derivative 1 | 5.0 | 0.3 |

*(iv) Blood sampling*

On Day 24, all mice were fasted for four hours and a 50 µl blood sample was collected from the tail vein into a heparinised tube for each mouse. Plasma was separated (yielding a 20 µl sample volume) and stored at -80 °C until analysis. Insulin was measured using the commercial MSD platform (Meso Scale Diagnostics), according to manufacturer's instructions.

*Results*

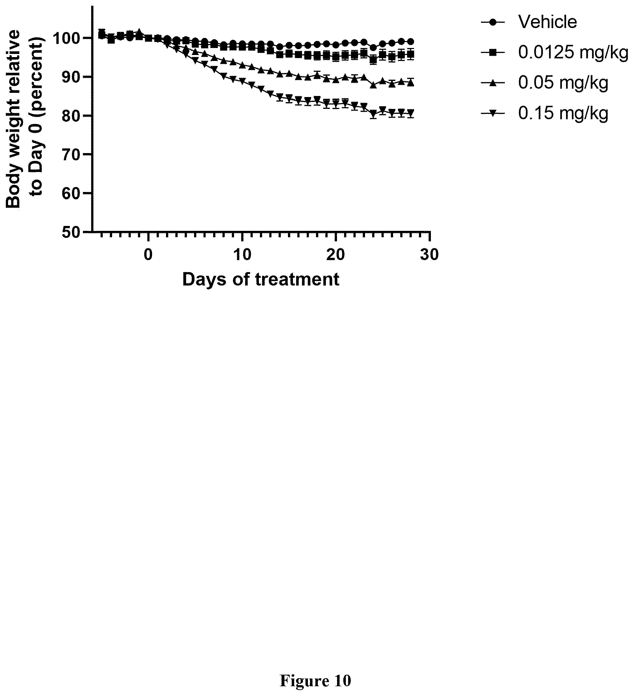

Figure 10 compares body weight over time for mice in Groups 1-4. The mice in Group 1 (administered only the PBS vehicle) were characterised by high body weight throughout the time course. A dose-dependent reduction in body weight was observed with treatment in Groups 2-4 (dosed with 0.0125 mg/kg, 0.05 mg/kg or 0.15 mg/kg Derivative 6) over time.

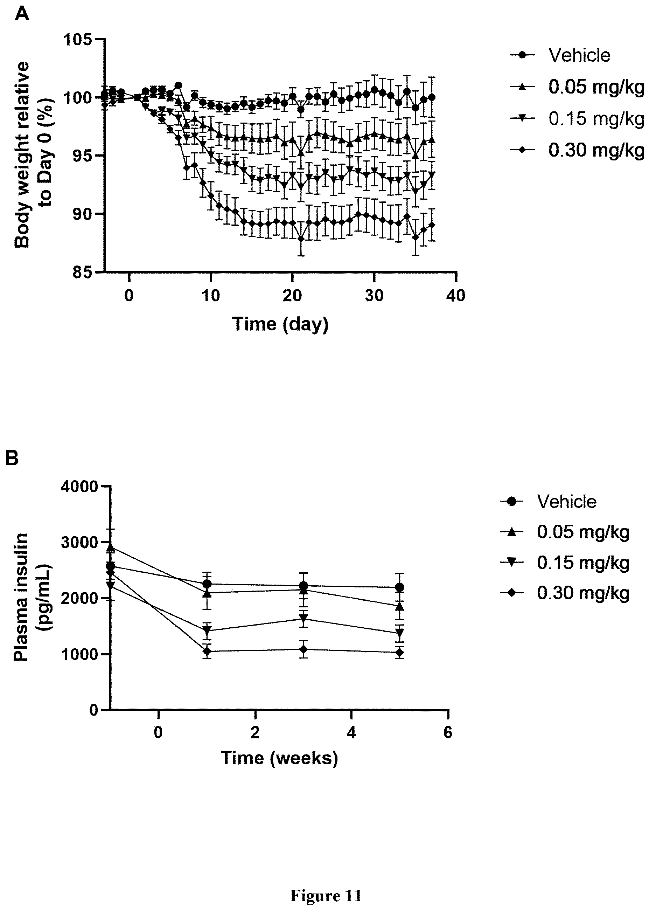

Figure 11A compares body weight over time for mice in Groups 1 and 5-7. As above, the mice in Group 1 (administered only the PBS vehicle) were characterised by high body weight throughout the time course. A dose-dependent reduction in body weight was again observed with treatment in Groups 5-7 (dosed with 0.05 mg/kg, 0.15 mg/kg or 0.3 mg/kg Derivative 1) over time.

Figure 11B compares fasting plasma insulin over time for mice in Groups 1 and 5-7. The mice in Group 1 (administered only the PBS vehicle) were characterised by high plasma insulin throughout the time course. A dose-dependent reduction in plasma insulin was observed with treatment in Groups 5-7 (dosed with 0.05 mg/kg, 0.15 mg/kg or 0.3 mg/kg Derivative 1) over time.

*Conclusions*

A reduction in body weight was achieved in a murine model of obesity after subcutaneous administration of Derivative 1 or Derivative 6. A reduction in plasma insulin was concomitantly achieved in the mice treated with Derivative 1. This supports the use of Derivative 1, 6 and other derivatives as described herein in the treatment of obesity in man. Steps are currently needed to tackle obesity in the global population because, as well as causing obvious physical changes, it can lead to a number of serious and potentially life-threatening conditions, such as type 2 diabetes, coronary heart disease and some types of cancer, such as breast cancer and bowel cancer.

**Example 8 - Therapeutic Effect in Liver Injury Mouse Model**

*Objective*

This study aimed to evaluate the effect of treatment with Derivative 1 in a Concanavalin A-induced liver immune-mediated injury mouse model.

*Methods*

*(i) Animals*

55 male six-week old pathogen-free C57B1/6J mice were acquired from Japan SLC, Inc. (Japan), housed *according to standard procedures* (up to five mice per cage) and fed a sterilised normal diet and drinking water *ad libitum.* Body weight was recorded daily prior to treatment. One day prior to study start, mice were randomised according to body weight into one group of five mice (Group 1) and five groups of 10 mice (Groups 2-6). Group 1 mice were kept without any treatment throughout the study period, to act as controls.

*(ii) Dose formulation preparation*

Derivative 1 was prepared as a 9.48 mg/mL stock solution in PBS. The dose formulations were prepared by diluting the stock solution in sterile filtered PBS to the required concentrations. PBS was used as a control vehicle and was prepared according to standard procedures.

*(iii) Treatment*

5 mL/kg of Derivative 1 or PBS was administered subcutaneously to the mice in Groups 2-6 at 72, 48, 24 and 1 h before Concanavalin A injection, as detailed in Table 13.

*Table 13: Groups of mice in study*

| Group | n | Mice | Formulation | Dose volume (mL/kg) | Dose (mg/kg) |
|---|---|---|---|---|---|
| 1 | 5 | Normal | None | n/a | n/a |
| 2 | 10 | Concanavalin A | Vehicle | 5.0 | n/a |
| 3 | 10 | Concanavalin A | Derivative 1 | 5.0 | 0.05 |
| 4 | 10 | Concanavalin A | Derivative 1 | 5.0 | 0.15 |
| 5 | 10 | Concanavalin A | Derivative 1 | 5.0 | 0.30 |
| 6 | 10 | Concanavalin A | Derivative 1 | 5.0 | 0.60 |

*(iv) Induction of hepatitis*

Liver immune-mediated injury (hepatitis) was induced in Group 2-6 mice by a single intravenous injection, at a dose of 20 mg/kg, of Concanavalin A (Sigma-Aldrich Co. LLC, USA) dissolved in saline at a concentration of 2.5 mg/mL.
Viability, clinical signs (lethargy, twitching, laboured breathing) and behaviour were monitored daily.
Mice were sacrificed 48 h after Concanavalin A injection by exsanguination through direct cardiac puncture under isoflurane anaesthesia.

*(v) Blood sampling*

Blood was collected at sacrifice into pre-cooled polypropylene tubes with anticoagulant and stored on ice until centrifugation. The blood samples were centrifuged at 1000 g for 15 minutes at 4 °C.

*(vi) Bioanalysis*

Plasma ALT levels were measured by FUJI DRI-CHEM 7000 (Fujifilm Corporation, Japan).
Statistical analysis was performed using Prism Software 6(GraphPad Software, USA). A Bonferroni Multiple Comparison was made between Group 2 (vehicle) and the remaining groups. P values of <0.05 were considered statistically significant. Results were expressed as mean $\pm$ standard deviation.

*Results*

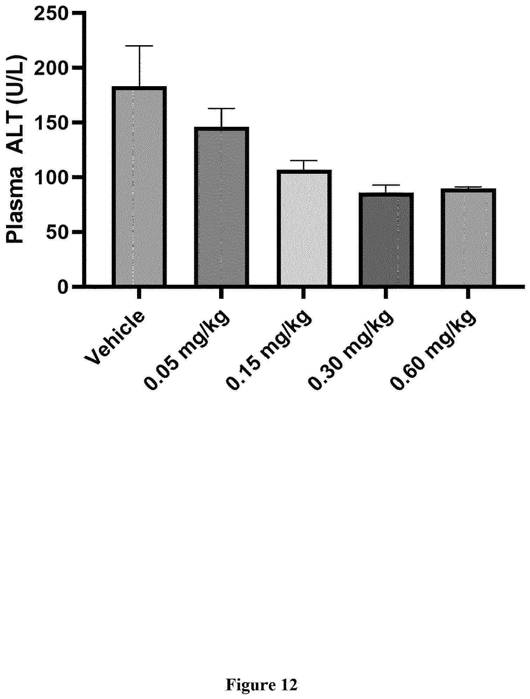

Figure 12 compares plasma ALT levels for mice in Groups 2-6. The mice in Group 2 (administered only the PBS vehicle) were characterised by plasma ALT. A dose-dependent reduction in plasma ALT was observed with treatment in Groups 3-6 (dosed with 0.05, 0.15, 0.30 or 0.60 mg/kg Derivative 1).

*Conclusions*

A reduction in plasma ALT levels was achieved in a murine model of liver immune-mediated injury after subcutaneous administration of Derivative 1. A beneficial effect was observed with a dose of just 0.05 mg/kg. The data hence support the use of Derivative 1 and other derivatives as described herein to prevent or mitigate liver immune-mediated injury (hepatitis).

**Example 9** - **Scaling Animal Data to Humans**

*Objective*

This study aimed to determine exposure to Derivative 1 after subcutaneous injection and intravenous infusion in humans, based on the minipig and mouse PK measures obtained in Examples 1, 2 and 4.

*Methods*

An allometrically scaled cross-species population PK model of Derivative 1 exposure as a function of dose, time and body weight was created based on the one-week single-dose data in minipigs described in Examples 1 and 2. Allometric scaling principles were applied with parameter values normalised to a body weight of 70 kg.

*Results*

Table 14 shows the average predicted PK parameters after subcutaneous and intravenous administration of Derivative 1 for a 70 kg human up to a week after single doses, as scaled from the PK data provided in Examples 1 and 2. PK parameters include the average concentration at t = 168 h ($C_{average168}$) and the AUC at t = 168 h ($AUC_{168}$).

*Table 14: PKparameters in humans (IV = intravenous; SC = subcutaneous)*

| Route | Dose | $C_{min}$ (ng/mL) | $C_{max}$ (ng/mL) | $C_{average168}$ (ng/mL) | $AUC_{168}$ (ng/mL*h) | $T_{max}$ (h) |
|---|---|---|---|---|---|---|
| IV | 1 μg/kg | 1.95 | 6.93 | 2.87 | 483 | 0.5 |
| IV | 3 μg/kg | 5.84 | 20.8 | 8.62 | 1450 | 0.5 |
| IV | 10 μg/kg | 19.5 | 69.3 | 28.7 | 4830 | 0.5 |
| IV | 30 μg/kg | 58.4 | 208 | 86.2 | 14500 | 0.5 |
| IV | 60 μg/kg | 117 | 416 | 172 | 29000 | 0.5 |
| IV | 100 μg/kg | 195 | 693 | 287 | 48300 | 0.5 |
| IV | 120 μg/kg | 234 | 832 | 345 | 57900 | 0.5 |
| IV | 200 μg/kg | 389 | 1390 | 575 | 96600 | 0.5 |
| IV | 300 μg/kg | 584 | 2080 | 862 | 145000 | 0.5 |
| SC | 1 μg/kg | 1.13 | 2.47 | 1.61 | 270 | 9.9 |
| SC | 3 μg/kg | 3.39 | 7.42 | 4.82 | 810 | 9.9 |
| SC | 10 μg/kg | 11.3 | 24.7 | 16.1 | 2700 | 9.9 |
| SC | 30 μg/kg | 33.9 | 74.2 | 48.2 | 8100 | 9.9 |
| SC | 60 μg/kg | 67.8 | 148 | 96.4 | 16200 | 9.9 |
| SC | 100 μg/kg | 113 | 247 | 161 | 27000 | 9.9 |
| SC | 120 μg/kg | 136 | 297 | 193 | 32400 | 9.9 |
| SC | 200 μg/kg | 226 | 494 | 321 | 54000 | 9.9 |
| SC | 300 μg/kg | 339 | 742 | 482 | 81000 | 9.9 |
| SC | 400 μg/kg | 452 | 989 | 643 | 108000 | 9.9 |

The lower bound of the dose range in humans was found to be 1 μg/kg for both subcutaneous injection and intravenous infusion. This was based on the first therapeutic effect observed in a murine acute DSS-induced colitis model administered Derivative 1 subcutaneously at a dose of 0.05 mg/kg (see Figure 7 and Example 3). Based on a single dose mouse PK study (Example 4), this dose results in a $C_{max}$ of 1.12 ng/mL and an $AUC_{INF}$ of 22.7 h*ng/mL in mice. Since 1 μg/kg is the lowest practical dose for Derivative 1, it therefore also is the lowest therapeutic dose.

The higher bound of the dose range was found to be 400 μg/kg for subcutaneous injection and 300 μg/kg for intravenous infusion. This was based on *in vitro* STAT3 activation in a IL-22 receptor reporter BHK cell line (Figure 8). Here the average $EC_{99}$ for two technical replicates of duplicate measures each was found to be 883 ng/mL (Table 10). The given doses are the highest doses for their respective route of administration during which the average exposure ($C_{average}$) does not exceed the value observed in the *in vitro* assay. As such, dosing higher would not be predicted to result in an increase in therapeutic efficacy.

*Conclusions*

The animal PK data were scaled to humans to predict exposures after subcutaneous or intravenous administration of Derivative 1 that allow setting of the therapeutic range.

**Example 10** - **Clinical Study Protocol**

*Objective*

To investigate safety, tolerability, PK, immunogenicity and exploratory PD of Derivative 1 following subcutaneous, or subcutaneous and intravenous, administration in healthy participants.

*Overview*

A Phase I randomised, double-blind, placebo-controlled, single centre, single and multiple ascending dose study of Derivative 1 in healthy adult volunteers. The primary route of administration was subcutaneous with the possibility to switch to a 30-minute intravenous infusion in case of unacceptable local tolerance at the injection site.

*Methods*

*(i) Study design*

The study population consisted of healthy volunteers, between 18 and 55 years old at screening, extremes included, and having a body weight in the range of 50 to 100 kg, extremes included, with a body mass index within the range of 18.5 to 27.0 kg/m2, extremes included, at screening.

A maximum of 96 participants (excluding replacements) received Derivative 1 or placebo at different escalating doses in two study parts: up to 64 participants in Part 1 (single ascending doses) and up to 32 participants in Part 2 (multiple ascending doses). Participants were not allowed to participate in more than one cohort.

Participants were screened over a period of up to 28 days. Eligible participants visited the clinical site in the morning of Day -1 for assessment of clinical laboratory tests, urinalysis, oral temperature and Coronavirus disease 2019 (COVID-19) testing. They were admitted to the clinical site in the evening of Day -1. Participants were discharged after performing the last assessment on Day 4 in Part 1 and Day 22 in Part 2 at the discretion of the investigator. Participants may have returned to the clinical site for ambulatory visits on Days 29, 36, 43, 50, 57 and 71.

The total duration of involvement for each participant, screening through follow-up, was approximately 12 weeks for Part 1 and 14 weeks for Part 2.

No interim analysis was performed.

Part 1 (single ascending dose) evaluated up to seven dose levels in a maximum of eight cohorts. Each cohort consisted of eight healthy participants who each received a single dose of either Derivative 1 (n = 6) or placebo (n = 2). The interval between participants dosed in subsequent cohorts was at least 14 days.

In each cohort, two sentinel participants received either Derivative 1 or placebo on the same day. The remainder of participants in a cohort were only dosed once safety data of at least 48 hours after dosing of the sentinel participants was reviewed. The two sentinel participants were randomised at a 1:1 ratio (Derivative 1:placebo) and the remaining six participants at a 5:1 ratio.

In Cohort 1, a single dose of 3 μg/kg of Derivative 1 or placebo was administered by subcutaneous injection in the abdomen or thigh. The following cohorts received Derivative 1 or placebo either subcutaneously (at 10-400 μg/kg) or intravenously (at 3-300 μg/kg). In the unexpected event that adverse skin reactions at the injection site led to a decision to switch to intravenous administration, Derivative 1 or placebo was administered as a 30-minute continuous intravenous infusion.

Table 15 provides an overview of dose levels for each cohort in Part 1 with and without a switch to a 30-minute intravenous infusion:

*Table 15: Dose levels for cohorts in Part 1 of the study*

| Cohort No. | Subcutaneous Dose Level (μg/kg) | Intravenous Dose Level (μg/kg) if Switch After Subcutaneous Cohort Number | | | | |
|---|---|---|---|---|---|---|
| | | 1 | 2 | 3 | 4 | 5 |
| 1 | 3 | - | - | - | - | - |
| 2 | 10 | 3 | - | - | - | - |
| 3 | 30 | 10 | 10 | - | - | - |
| 4 | 100 | 30 | 30 | 30 | - | - |

(continued)

| Cohort No. | Subcutaneous Dose Level (μg/kg) | Intravenous Dose Level (μg/kg) if Switch After Subcutaneous Cohort Number | | | | |
|---|---|---|---|---|---|---|
| | | 1 | 2 | 3 | 4 | 5 |
| 5 | 200 | 100 | 100 | 100 | 30 | - |
| 6 | 300 | 200 | 200 | 200 | 100 | 100 |
| 7 | 400 | 300 | 300 | 300 | 200 | 200 |
| 8 | - | - | - | - | 300 | 300 |

The first intravenous dose after switching from subcutaneous was designed to achieve an exposure that did not exceed the expected exposure (considering both $C_{max}$ and $AUC_{0-168h}$) of the next planned subcutaneous dose.

Start of Part 2 and the dose (including the loading dose) for the first cohort in Part 2 was decided based on a review of available blinded safety and tolerability data up to at least Day 8 from Cohort 5 in Part 1 and blinded PK data up to at least Day 8 from Cohorts 1 to 4 in Part 1. In case Part 2 was conducted with intravenous administration, PK data up to at least Day 8 from at least one intravenous single ascending dose cohort in Part 1 were included. All available biomarker data were also taken into account, and the decision was supported by modeling and simulation predictions.

Part 2 (multiple ascending dose) evaluated up to four dose levels in a maximum of four cohorts. Each cohort consisted of eight healthy participants who each received three doses of either Derivative 1 or placebo on Days 1, 8 and 15. Participants were randomised at a 6:2 ratio (Derivative Eplacebo). The interval between the first participants dosed in Cohort 1 and Cohort 2 was least 35 days. The interval between the first participants dosed in subsequent cohorts was at least 28 days.

For each cohort, the first dose on Day 1 was a loading dose that was higher than the doses administered on Day 8 and Day 15 with the intention to achieve steady state after the first dose of Derivative 1. The dose did not exceed the maximum dose of 400 μg/kg subcutaneously or 300 μg/kg intravenously. Specifically, the dosing schedule was as follows. Cohort 1: a loading dose of 2.5 μg/kg body weight followed by a weekly dose of 1.25 μg/kg body weight (loading dose given in the first week and the weekly doses for the rest of the treatment period). Cohort 2: a loading dose of 5 μg/kg followed by a 2.5 μg/kg weekly dose. Cohort 3: a loading dose of 10 μg/kg followed by 5 μg/kg weekly dose. Cohort 4: a loading dose of 15 μg/kg followed by 7.5 μg/kg weekly dose. Administration of Derivative 1 or placebo was either subcutaneous or intravenous, depending on whether a decision was taken to switch to intravenous administration in Part 1.

Note that a subcutaneous dose level of 3 μg/kg body weight was found to be more potent than expected in the study. Accordingly a subcutaneous dose level of 1 μg/kg body weight was additionally included.

*(ii) Dose formulation*

Derivative 1 was provided in 2 mL vials with 1 mL extractable volume for single use. The unit dose strength was 10.1 mg/mL.

Placebo control was used to establish the frequency and magnitude of changes in clinical endpoints that may occur in the absence of active test product. The placebo comparator for subcutaneous administration was a non-preserved sterile ready to use liquid formulation without active product with pH = 7.0, provided in 2 mL vials with 1 mL extractable volume for single use. The placebo comparator for intravenous use was normal saline (0.9 g/L sodium chloride), provided in a bottle for intravenous infusion.

*(iii) Assessments*

Blood samples were collected for PK analysis of Derivative 1 in serum.

The following PK parameters were determined for Derivative 1 by non-compartmental analysis using the individual serum concentration-time profiles, with actual sampling times:

For Part 1: $C_{max}$, $t_{max}$, $AUC_{0-168h}$, $AUC_{last}$, $AUC_{inf}$, apparent first order terminal rate constant ($\lambda z$), and $t_{1/2}$. In case of intravenous administration, also CL, Vz and estimated distribution volume at steady state (Vss) were calculated.

For Part 2 (after each dose): $C_{max}$, $t_{max}$, average serum concentration over the dosing interval ($C_{avg}$), trough serum concentration observed at the end of the dosing interval ($C_{trough}$), $AUC_{0-168h}$ (i.e., $AUC_{tau}$ at steady state), $AUC_{inf}$

(only after the last dose), $\lambda z$, $t_{1/2}$ and accumulation ratio (Rac calculated from dose-corrected values for $C_{max}$ and $AUC_{0\text{-}168h}$ (both after second and third dose over the first dose). In case of intravenous administration, also CL, Vz and Vss (only after the last dose) were calculated.

Serum and plasma samples for the determination of a seven-plex cytokine panel and various biomarkers were obtained from blood collected at the following time points during Part 1 of the study:

*Table 16: Time points for blood collection in Part 1 of the study (X = required; O = optional)*

| Assessments | Time of visit (days) | Serum sample for cytokine panel | Serum sample for biomarkers | Plasma sample for biomarker |
|---|---|---|---|---|
| Intervention period | D1 predose | X | X | X |
| | D1 postdose | X | X | X |
| | D2 | X | X | X |
| | D3 | X | X | - |
| | D4 | X | X | X |
| | D5 | - | X | - |
| | D6 | - | X | - |
| | D8 | - | X | X |
| | D11±1 | - | X | - |
| | D15±1 | - | X | - |
| | D22±2 | - | X | X |
| | D29±2 | - | X | - |
| | D36±2 | - | X | - |
| | D43±2 | - | X | X |
| Follow-up/ withdrawal | D57±3 | - | X | X |
| Unschedule | - | O | O | O |

Serum and plasma samples for the determination of the same cytokines and biomarkers were obtained from blood collected at the following time points during Part 2 of the study:

*Table 17: Time points for blood collection in Part 2 of the study (X = required; O = optional)*

| Assessments | Time of visit (days) | Serum sample for cytokine panel | Serum sample for biomarkers | Plasma sample for biomarker |
|---|---|---|---|---|
| Intervention period | D1 predose | X | X | X |
| | D1 postdose | X | X | X |
| | D2 | X | X | - |
| | D3 | X | X | - |
| | D5 | - | - | X |
| | D8 predose | X | X | X |
| | D8 postdose | X | X | X |

(continued)

| Assessments | Time of visit (days) | Serum sample for cytokine panel | Serum sample for biomarkers | Plasma sample for biomarker |
|---|---|---|---|---|
| | D9 | X | X | - |
| | D10 | X | X | - |
| | D12 | - | - | X |
| | D15 predose | X | X | X |
| | D15 postdose | X | X | X |
| | D16 | X | X | - |
| | D17 | X | X | - |
| | D19 | - | - | X |
| | D22 | - | - | X |
| | D26±2 | - | X | - |
| | D29±2 | - | X | - |
| | D36±2 | - | X | - |
| | D43±2 | - | X | - |
| | D50±2 | - | X | - |
| | D57±2 | - | X | - |
| Follow-up/ withdrawal | D71±3 | - | X | X |
| Unschedule | - | O | O | O |

Blood samples were kept deep frozen during shipment and stored at -75 °C ± 10 °C until analysis.

The PD parameters measured were absolute values and changes from baseline in: serum levels of the biomarkers, REG3a and hsCRP, and SAA; plasma levels of the biomarker fibrinogen-C; serum levels of a cytokine panel (including interferon [IFN]-$\gamma$, tumor necrosis factor [TNF]-$\alpha$, IL-1$\beta$, IL-2, IL-6, IL 8, and IL-10).

Cytokine and SAA analysis in serum was performed using an electrochemiluminescence immunoassay (SGS France) with a commercial kit (Meso Scale Discovery) according to manufacturer's instructions. Analysis of the serum samples for the determination of REG3A was performed using an Ella platform with a commercial ProteinSimple Human REG3A Simple Plex assay cartridge according to manufacturer's instructions (Bio-Techne, USA). Analysis of the serum samples for the determination of hsCRP was performed by immunoturbidimetric assay on latex particles using a commercial Cardiac C-reactive Protein (Latex) High Sensitive pack according to manufacturer's instructions (Roche Diagnostics GmbH, USA). All samples were assayed directly by the Cobas® 6000 platform, in duplicate for calibration standards and in singulate for control and clinical samples. Analysis of the plasma samples for the determination of fibrinogen-C was performed using an ELISA assay with a commercial Human Fibrinogen ELISA kit according to manufacturer's instructions (Aviva Systems Biology, USA).

The immunogenicity of Derivative 1 was evaluated by assessing the presence, specificity, and titer of anti-drug antibodies.

The study evaluated safety and tolerability through assessment of adverse events, skin and injection site inspection, clinical laboratory tests, 12-lead electrocardiogram, vital signs and physical examination.

Statistical evaluation used standard statistical tools (such as arithmetic mean, standard deviation, median, minimum, maximum, coefficient of variation [CV%], geometric mean and geometric CV%).

*Results*

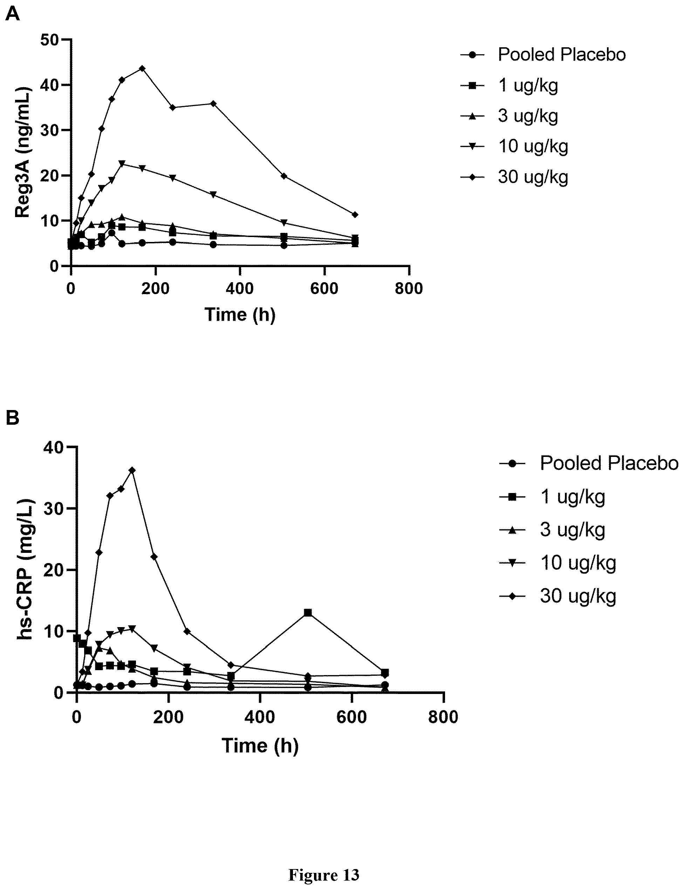

Figure 13A compares REG3A levels over time following a single subcutaneous administration of Derivative 1 in humans. Data from cohorts receiving placebo (a non-preserved sterile ready to use liquid formulation without active product with pH = 7.0) were pooled. These subjects demonstrated baseline levels of REG3A during the time course, which included a peak response at around 100 h. A dose-dependent increase in REG3A levels was observed on treatment with Derivative 1 (dosed at 1-30 μg/kg). The peak response was progressively greater with increasing dose and lasted longer than at baseline. All doses achieved a REG3A response above baseline levels.

Figure 13B compares hsCRP levels over time following a single subcutaneous administration of Derivative 1 in humans. Data from cohorts receiving the placebo were pooled. These subjects demonstrated baseline levels of hsCRP during the time course. A dose-dependent increase in hsCRP levels was observed on treatment with Derivative 1 (dosed at 1-30 μg/kg). The peak response was progressively greater with increasing dose and lasted longer than at baseline. All doses achieved a hsCRP response above baseline levels.

*Conclusions*

An increase in REG3A and hsCRP was achieved in humans after subcutaneous administration of Derivative 1 (dosed at 1-30 μg/kg). REG3A and hsCRP are biomarkers demonstrating IL-22 target engagement in the liver and intestine. As such, the data show that Derivative 1 was able to bind IL-22 receptors and engage the different activities/pathways that are responsible for IL-22-mediated beneficial effects. No such biomarker response has been shown by competitor products. Moreover, all of the tested doses achieved REG3 A and hsCRP responses above baseline levels. As little as 1 μg/kg of Derivative 1 was therefore shown to have a beneficial effect. This potency of Derivative 1 in man was completely surprising. The data hence support the therapeutic use of derivatives as described herein in man, even at low doses.

While certain features of the invention have been illustrated and described herein, many modifications and equivalents will occur to those of ordinary skill in the art. It is, therefore, to be understood that the claims are intended to cover all such modifications and equivalents as fall within the true spirit of the invention.

**Claims**

1. A derivative of IL-22 comprising a fatty acid covalently attached to an IL-22 protein, for use in a method of treating a metabolic, gut and/or liver disease, disorder or condition, said method comprising administering the derivative of IL-22 subcutaneously.

2. A derivative of IL-22 comprising a fatty acid covalently attached to an IL-22 protein, for use in a method of treating a metabolic, gut and/or liver disease, disorder or condition, said method comprising administering the derivative of IL-22 intravenously.

3. A derivative for use in a method as claimed in either of the preceding claims, wherein the fatty acid is:

   (i) a C12, C14, C16, C18 or C20 diacid;
   (ii) a C14, C16 or C18 diacid; and/or
   (iii) a C18 diacid.

4. A derivative for use in a method as claimed in any of the preceding claims, wherein the IL-22 protein is native mature human IL-22 (hIL-22; SEQ ID NO. 1) or a variant thereof.

5. A derivative for use in a method as claimed in claim 4, wherein the variant:

   (i) is substituted at position 1, 6, 33, 35, 64, 95 and/or 106 of hIL-22;
   (ii) comprises a substitution of hIL-22 selected from the group consisting of A1C, H6C, A33C, N35Q, N64Q, R95C and L106C;
   (iii) comprises a Cys residue at position 1 of hIL-22;
   (iv) comprises a Cys residue at position 95 of hIL-22;
   (v) comprises a Cys residue at position 106 of hIL-22;
   (vi) has at least 10% sequence identity with hIL-22; and/or
   (vii) comprises one, two, three, four, five or more variations within hIL-22, wherein said variations are independently selected from the group consisting of deletions, substitutions and insertions.

6. A derivative for use in a method as claimed in claim 4 or claim 5, wherein the variant:

(i) comprises an N-terminal peptide;
(ii) comprises an N-terminal trimer;
(iii) comprises an N-terminal G;
(iv) comprises an N-terminal G-P-G;
(v) comprises an N-terminal peptide of up to five, 10, 15, 20, 25, 30, 35, 40, 45 or 50 amino acids.

7. A derivative for use in a method as claimed in any of the preceding claims, wherein the fatty acid is covalently attached to the IL-22 protein by a linker, the linker comprising:

(i) one or more amino acids, optionally including Glu and/or Lys;
(ii) one or more oligo(ethylene glycol) (OEG) residues;
(iii) an ethylenediamine ($C_2$DA) group;
(iv) an acetamide (Ac) group;
(v) $\gamma$Glu-OEG-OEG-$C_2$DA-Ac; and/or
(vi) $\gamma$Glu-$\gamma$Glu-$\gamma$Glu-$\gamma$Glu-OEG-OEG-$\epsilon$Lys-$\alpha$Ac.

8. A derivative for use in a method as claimed in claim 7, wherein the linker is a Cys-reactive linker attached to a Cys residue:

(i) in the hIL-22 or variant thereof;
(ii) substituted at position 1, 6, 33, 95 or 106 of hIL-22;
(iii) at position -7 relative to hIL-22;
(iv) substituted at position 1 of hIL-22;
(v) substituted at position 95 of hIL-22; and/or
(vi) substituted at position 106 of hIL-22.

9. A derivative for use in a method as claimed in any of the preceding claims, wherein the derivative comprises a C18 diacid covalently attached by a linker to a variant of hIL-22, wherein the variant comprises an N-terminal G-P-G and a Cys residue substituted at position 1 of hIL-22 and the linker is attached to said Cys residue.

10. A derivative for use in a method as claimed in any of claims 1-8, wherein the derivative comprises a C18 diacid covalently attached by a linker to a variant of hIL-22, wherein the variant comprises a Cys residue substituted at position 95 or 106 of hIL-22 and the linker is attached to said Cys residue.

11. A derivative for use in a method as claimed in any of the preceding claims, wherein the derivative is Derivative 1-14 as identified herein.

12. A derivative for use in a method as claimed in any of the preceding claims, wherein:

(i) the metabolic disease, disorder or condition is obesity, diabetes type 1, diabetes type 2, hyperlipidemia, hyperglycemia or hyperinsulinemia;
(ii) the liver disease, disorder or condition is non-alcoholic fatty liver disease (NAFLD), non-alcoholic steatohepatitis (NASH), cirrhosis, alcoholic hepatitis, acute liver failure, chronic liver failure, acute-on-chronic liver failure (ACLF), acetaminophen induced liver toxicity, acute liver injury, sclerosing cholangitis, biliary cirrhosis or a pathological condition caused by surgery or transplantation; or
(iii) the gut disease, disorder or condition is inflammatory bowel disease (IBD), ulcerative colitis, Crohn's disease, graft-versus-host-disease (GvHD), a chemical injury, a viral infection, a bacterial infection or short bowel disease.

13. A derivative for use in a method as claimed in any of the preceding claims, wherein the method comprises administering the derivative of IL-22 subcutaneously by injection or intravenously by infusion.

14. A derivative for use in a method as claimed in any of claims 1 and 3-13, wherein the method comprises administering the derivative of IL-22 subcutaneously in a weekly dose of between approximately:

(i) 1 $\mu$g/kg and 500 $\mu$g/kg;
(ii) 1 $\mu$g/kg and 15 $\mu$g/kg;
(iii) 3 $\mu$g/kg and 100 $\mu$g/kg;
(iv) 5 $\mu$g/kg and 80 $\mu$g/kg;

(v) 5 μg/kg and 15 μg/kg;
(vi) 10 μg/kg and 150 μg/kg;
(vii) 20 μg/kg and 150 μg/kg;
(viii) 25 μg/kg and 300 μg/kg;
(ix) 30 μg/kg and 150 μg/kg;
(x) 50 μg/kg and 350 μg/kg;
(xi) 100 μg/kg and 400 μg/kg; and/or
(xii) 200 μg/kg and 400 μg/kg;

of body weight.

15. A derivative for use in a method as claimed in any of claims 2-13, wherein the method comprises administering the derivative of IL-22 intravenously in a weekly dose of between approximately:

(i) 1 μg/kg and 400 μg/kg;
(ii) 1 μg/kg and 15 μg/kg;
(iii) 3 μg/kg and 100 μg/kg;
(iv) 5 μg/kg and 80 μg/kg;
(v) 5 μg/kg and 15 μg/kg;
(vi) 10 μg/kg and 150 μg/kg;
(vii) 20 μg/kg and 150 μg/kg;
(viii) 25 μg/kg and 300 μg/kg;
(ix) 30 μg/kg and 150 μg/kg;
(x) 50 μg/kg and 350 μg/kg;
(xi) 100 μg/kg and 300 μg/kg; and/or
(xii) 150 μg/kg and 300 μg/kg;

of body weight.

A

B

C

**Figure 1**

-3 GPGCPISSHCRLDKSNFQQPYITNRTFMLAKEASLADNNTDVRLIGEKLFHGVS

52 MSERCYLMKQVLNFTLEEVLFPQSDRFQPYMQEVVPFLARLSNRLSTCHIEGDD

106 LHIQRNVQKLKDTVKKLGESGEIKAIGELDLLFMSLRNACI

**Figure 2**

-3 G P G C P I S S H C R L D K S N F Q Q P Y I T N R T F M L A K E A S L A D Q N T D V R L I

43 G E K L F H G V S M S E R C Y L M K Q V L Q F T L E E V L F P Q S D R F Q P Y M Q E V V P

88 F L A R L S N R L S T C H I E G D D L H I Q R N V Q K L K D T V K K L G E S G E I K A I G

133 E L D L L F M S L R N A C I

Figure 3

-3 G P G C P I S S H C R L D K S N F Q Q P Y I T N R T F M L A K E A S L A D Q N T D V R L I G E K L F H G V S

52 M S E R C Y L M K Q V L Q F T L E E V L F P Q S D R F Q P Y M Q E V V P F L A R L S N R L S T C H I E G D D

106 L H I Q R N V Q K L K D T V K K L G E S G E I K A I G E L D L L F M S L R N A C L,

Figure 4

Figure 5

Figure 6

**Figure 7**

**Figure 8**

44

Figure 9

Figure 10

Figure 11

Figure 12

Figure 13

Europäisches Patentamt
European Patent Office
Office européen des brevets

# EUROPEAN SEARCH REPORT

Application Number

EP 23 17 9636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/347304 A1 (SASS-ØRUM KRISTIAN [DK] ET AL) 3 November 2022 (2022-11-03) | 1-13 | INV.<br>A61K38/20 |
| Y | * figures 5, 6a; sequence 16 *<br>* examples 3-4 *<br>* example 8 *<br>* paragraph [0228] - paragraph [0233] *<br>* claims 1-18 *<br>* paragraph [0113] - paragraph [0114] *<br>----- | 14,15 | A61P1/16<br>A61P3/04<br>A61P3/10<br>A61K47/54 |
| Y | WO 2021/212056 A2 (UNIV LELAND STANFORD JUNIOR [US]) 21 October 2021 (2021-10-21) | 14,15 | |
| A | * example 6 *<br>* claims 1-81 *<br>* paragraph [0182] - paragraph [0185] *<br>----- | 1-13 | |
| Y | EP 4 089 107 A1 (CYTOKI PHARMA APS [DK]) 16 November 2022 (2022-11-16)<br>* the whole document, especially paragraph 121-122 *<br>----- | 1-15 | |
| Y | EP 4 089 108 A1 (CYTOKI PHARMA APS [DK]) 16 November 2022 (2022-11-16)<br>* the whole document *<br>----- | 1-15 | **TECHNICAL FIELDS SEARCHED (IPC)**<br><br>A61K<br>A61P |

The present search report has been drawn up for all claims

| Place of search | Date of completion of the search | Examiner |
|---|---|---|
| Munich | 26 October 2023 | Steinheimer, K |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-15(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

**EP 23 17 9636**

The Search Division considers that the present European patent application does not comply with the requirements of unity of invention and relates to several inventions or groups of inventions, namely:

```
1. claims: 1-15(partially)

    A derivative of IL-22 comprising a fatty acid covalently
    attached to an IL-22 protein, for use in a method of
    treating a metabolic, gut and/or liver disease, disorder or
    condition, said method comprising administering the
    derivative of IL-22 subcutaneously, whereby the derivative
    is Derivative 1.
                        ---


2-14. claims: 1-15(partially)

    A derivative of IL-22 comprising a fatty acid covalently
    attached to an IL-22 protein, for use in a method of
    treating a metabolic, gut and/or liver disease, disorder or
    condition, said method comprising administering the
    derivative of IL-22 subcutaneously, whereby the derivative
    is Derivative 2-14, respectively.
    (It is noted that from these goups, only Derivative 6 was
    studied in the experiments. The medical use of the other
    agents that are claimed is not sufficiently disclosed (Art.
    5 PCT).)
                        ---
```

## ANNEX TO THE EUROPEAN SEARCH REPORT
## ON EUROPEAN PATENT APPLICATION NO.

EP 23 17 9636

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2023

| Patent document cited in search report | | | Publication date | Patent family member(s) | | | Publication date |
|---|---|---|---|---|---|---|---|
| US 2022347304 | A1 | | 03-11-2022 | AU | 2020378648 | A1 | 23-06-2022 |
| | | | | BR | 112022008497 | A2 | 26-07-2022 |
| | | | | CA | 3160338 | A1 | 14-05-2021 |
| | | | | CL | 2022001185 | A1 | 03-02-2023 |
| | | | | CN | 114761422 | A | 15-07-2022 |
| | | | | CO | 2022005889 | A2 | 29-07-2022 |
| | | | | CR | 20220249 | A | 19-08-2022 |
| | | | | EP | 3819307 | A1 | 12-05-2021 |
| | | | | EP | 4055035 | A1 | 14-09-2022 |
| | | | | IL | 292657 | A | 01-07-2022 |
| | | | | JP | 2023083380 | A | 15-06-2023 |
| | | | | JP | 2023502005 | A | 20-01-2023 |
| | | | | KR | 20220097921 | A | 08-07-2022 |
| | | | | PE | 20221279 | A1 | 05-09-2022 |
| | | | | US | 2022347304 | A1 | 03-11-2022 |
| | | | | US | 2023381320 | A1 | 30-11-2023 |
| | | | | WO | 2021089875 | A1 | 14-05-2021 |
| WO 2021212056 | A2 | | 21-10-2021 | AU | 2021257348 | A1 | 03-11-2022 |
| | | | | BR | 112022020796 | A2 | 29-11-2022 |
| | | | | CA | 3180108 | A1 | 21-10-2021 |
| | | | | CN | 115955976 | A | 11-04-2023 |
| | | | | EP | 4136101 | A2 | 22-02-2023 |
| | | | | IL | 297187 | A | 01-12-2022 |
| | | | | JP | 2023521867 | A | 25-05-2023 |
| | | | | KR | 20230004645 | A | 06-01-2023 |
| | | | | US | 2023192794 | A1 | 22-06-2023 |
| | | | | WO | 2021212056 | A2 | 21-10-2021 |
| EP 4089107 | A1 | | 16-11-2022 | NONE | | | |
| EP 4089108 | A1 | | 16-11-2022 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019101888 A
- WO 2022238503 A
- WO 2022238510 A

- WO 2021089875 A

**Non-patent literature cited in the description**

- **STEFANICH et al.** *Biochem Pharmacol,* 2018, vol. 152, 224-235

- **WAGNER F. et al.** *Gut,* 2023, vol. 0, 1-11